# EUROPEAN PATENT APPLICATION

(11) **EP 4 818 210 A1**
(43) Date of publication of application: **07.10.2026**
(21) Application number: 24896781.2
(22) Date of filing: 02.12.2024
(51) Int. Cl.: C07K 16/30, C07K 16/28, A61K 39/395, A61P 35/00

(54) **ANTIGEN-BINDING PROTEIN AND USE THEREOF**

(30) Priority: 01.12.2023 CN 202311639416
(71) Applicant: Minghui Pharmaceutical (Hangzhou) Ltd., Hangzhou, Zhejiang 310018 (CN); Minghui Pharmaceutical (Shanghai) Limited, Shanghai 201210 (CN)
(72) Inventor: LIU, Bo, Hangzhou, Zhejiang 310018 (CN); ZHU, Liyuan, Shanghai 201210 (CN); CAO, Guoqing, Shanghai 201210 (CN); SHI, Junwei, Shanghai 201210 (CN); WENG, Xialian, Hangzhou, Zhejiang 310018 (CN); CAO, Yuting, Hangzhou, Zhejiang 310018 (CN)
(74) Representative: Ipsilon
(86) International application number: PCT/CN2024/136085
(87) International publication number: WO 2025/113702

(57) **Abstract**

A nanobody specifically binding to HER2 or an antigen-binding fragment thereof, a composition comprising the nanobody or the antigen-binding fragment thereof, and a nucleic acid molecule encoding the antibody or the antigen-binding fragment thereof. In addition, the present invention further relates to the use of the nanobody or the antigen-binding fragment thereof in the treatment and diagnosis of diseases.

## Description

### Cross-Reference to Related Application

The present application claims priority to the Chinese patent application with application number CN 202311639416.3 (filed on December 1, 2023). The contents of the patent application are incorporated into the present application in their entirety by reference.

### Technical Field

The present invention relates to a nanobody or antigen-binding fragment thereof capable of specifically binding to HER2, a composition containing the nanobody or antigen-binding fragment thereof, and a nucleic acid molecule encoding the antibody or antigen-binding fragment thereof. Furthermore, the present invention relates to the use of the nanobody or antigen-binding fragment thereof in the treatment and diagnosis of diseases.

### Background

HER2, or human epidermal growth factor receptor-2, also known as receptor tyrosine kinase ErbB-2, is a 185 kDa receptor tyrosine kinase (RTK) in the epidermal growth factor receptor (EGFR) family. HER2 forms homodimers or heterodimers with three other members of the EGFR family (HER1 (EGFR, ErbB-1), HER3 (ErbB-3), and HER4 (ErbB-4)), leading to phosphorylation of receptor tyrosine residues. This, in turn, activates various signaling pathways related to cell proliferation and tumor development, such as the PI3K-AKT-mTOR pathway. Currently, no ligands have been found to directly bind to HER2. Furthermore, ErbB2 can reduce the expression of cyclin D and c-myc, which sequester the cyclin-dependent kinase (CDK) inhibitor p27kip1, preventing it from inhibiting CDK2 activity and ultimately leading to increased cell proliferation.

With expanding and deepening research, HER2 has been found to be expressed and overexpressed in various types of tumors. To date, HER2 overexpression has been reported in multiple cancer types, including breast cancer, gastric cancer, lung cancer, colorectal cancer, and ovarian cancer, and HER2 overexpression is associated with a poor prognosis in breast cancer. Therefore, there is an urgent clinical need for effective HER2-targeting drugs for the treatment of malignant tumors. Currently, trastuzumab and pertuzumab are the HER2-targeting monoclonal antibodies available on the market.

Trastuzumab is a humanized monoclonal antibody targeting HER2, developed by Genentech, Inc. in the United States (patent numbers: US5677171A and US5821337A). Trastuzumab exerts its antitumor activity by specifically binding to the extracellular domain IV of HER2, inducing antibody-dependent cellular cytotoxicity (ADCC), and inhibiting HER2-mediated signaling. It demonstrates strong therapeutic efficacy against tumors that overexpress HER2. In 1998, the FDA approved trastuzumab in combination with paclitaxel for the first-line treatment of patients with HER2-overexpressing advanced breast cancer, and as a single agent for the treatment of patients with HER2-overexpressing metastatic breast cancer who have received at least one prior course of chemotherapy.

Pertuzumab is also developed by Genentech, Inc. in the United States (patent number US7862817B2). Pertuzumab binds to the extracellular domain II of HER2 receptor, inhibiting dimer formation and thus suppressing receptor-mediated signal transduction pathways. In 2012, the US FDA approved pertuzumab in combination with trastuzumab and docetaxel for the treatment of patients with HER2-positive advanced metastatic breast cancer.

Over the past 20 years, HER2-targeted therapies have significantly improved the prognosis of patients with HER2-overexpressing breast cancer. However, only about 15% of breast cancer patients overexpress HER2, while the remaining approximately 85% patients with HER2-low or HER2-negative breast cancer are in urgent need of more effective HER2-targeted therapies. Additionally, HER2-positive breast cancer is prone to metastasis, particularly to the brain. Conventional antibodies have a molecular weight of approximately 150 kDa. Single-domain antibodies (nanobodies) derived from alpacas, when fused with human Fc, have a molecular weight of only about 75 kDa, half that of conventional antibodies. Their smaller structure, compared to standard monoclonal antibodies, enables enhanced penetration of the blood-brain barrier, allowing them to reach deeper regions of tumors. This maximizes the opportunity for antibody drugs to bind to tumor cells, leading to improved diagnostic and therapeutic outcomes.

Therefore, providing a single-domain antibody targeting HER2 holds promising potential for the diagnosis and treatment of tumors.

### Contents of the Invention

The inventors of the present application, through extensive research, have screened and obtained a series of anti-HER2 nanobodies and polypeptide constructs, which exhibit high binding activity to HER2, and do not compete with Trastuzumab or Pertuzumab for binding to the HER2 antigen. In particular, the nanobody or polypeptide construct of the present invention possess internalization activity, enabling them to mediate the entry of toxins into cells through internalization, thereby exerting a cytotoxic effect on tumor cells. On this basis, humanized nanobodies or polypeptide constructs prepared from these nanobodies retain these remarkable activities. Furthermore, the nanobodies or polypeptide constructs are characterized by their small molecular weight and ease of production.

Based on this, the present application further provides a conjugate containing the nanobody or antigen-binding fragment thereof, a nucleic acid molecule encoding the nanobody or antigen-binding fragment thereof, a host cell containing it, and its related uses.

### Nanobody or antigen-binding fragment thereof

In one aspect, the present invention provides a nanobody or antigen-binding fragment thereof capable of specifically binding to HER2. The nanobody described herein typically consists of 4 framework regions (FRs) and 3 complementarity-determining regions (CDRs), referred to as FR1, CDR1, FR2, CDR2, FR3, CDR3, and FR4, wherein the antigen-binding fragment comprises at least a portion of the nanobody, wherein the portion is sufficient to confer the ability to specifically bind HER2 to the fragment. In some embodiments, the nanobody described in the present invention may be truncated at the N-terminus or C-terminus to contain only a portion of FR1 and/or FR4, or to lack one or both of those framework regions, as long as it substantially retains antigen binding ability and specificity.

In some embodiments, the nanobody or antigen-binding fragment thereof comprises CDR1, CDR2, and CDR3 contained in a VHH as set forth in any one of SEQ ID NOs: 15-18.

In some embodiments, the CDRs are defined according to the Kabat, Chothia, or IMGT numbering system.

In some embodiments, the nanobody or antigen-binding fragment thereof comprises: CDR1 as set forth in SEQ ID NO: 1; CDR2 as set forth in SEQ ID NO: 2 or 3; and CDR3 as set forth in SEQ ID NO: 4; wherein the CDRs are defined according to the Kabat numbering system.

In some embodiments, the nanobody or antigen-binding fragment thereof comprises: CDR1 as set forth in SEQ ID NO: 5; CDR2 as set forth in SEQ ID NO: 6; and CDR3 as set forth in SEQ ID NO: 7; wherein the CDRs are defined according to the IMGT numbering system.

In some embodiments, the nanobody or antigen-binding fragment thereof comprises: CDR1 as set forth in SEQ ID NO: 8; CDR2 as set forth in SEQ ID NO: 9 or 10; and CDR3 as set forth in SEQ ID NO: 11; wherein the CDRs are defined according to the Kabat numbering system.

In some embodiments, the nanobody or antigen-binding fragment thereof comprises: CDR1 as set forth in SEQ ID NO: 12; CDR2 as set forth in SEQ ID NO: 13; and CDR3 as set forth in SEQ ID NO: 14; wherein the CDRs are defined according to the IMGT numbering system.

In some embodiments, the nanobody or antigen-binding fragment thereof comprises a frame region sequence of a camelid antibody.

In some embodiments, the nanobody or antigen-binding fragment thereof comprises the sequence as set forth in SEQ ID NO: 15, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or more amino acids (e.g., a substitution, deletion, or addition of 1, 2, 3, 4, or 5 amino acids) as compared thereto. In some embodiments, the nanobody or antigen-binding fragment thereof comprises the sequence as set forth in SEQ ID NO: 15.

In some embodiments, the nanobody or antigen-binding fragment thereof comprises the sequence as set forth in SEQ ID NO: 17, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or more amino acids (e.g., a substitution, deletion, or addition of 1, 2, 3, 4, or 5 amino acids) as compared thereto. In some embodiments, the nanobody or antigen-binding fragment thereof comprises the sequence as set forth in SEQ ID NO: 17.

In some embodiments, the nanobody or antigen-binding fragment thereof is humanized, i.e., one or more of its frame regions have been substantially replaced by human frame regions.

In some embodiments, the nanobody or antigen-binding fragment thereof further comprises a heavy chain framework region of a human immunoglobulin (e.g., a heavy chain framework region comprised in an amino acid sequence encoded by a human heavy chain germline antibody gene), the heavy chain framework region optionally comprising one or more (e.g., 1, 2, 3, 4, 5, 6, 7, 8, 9, or 10) back mutations from human residues to camelid residues.

In some embodiments, the nanobody or antigen-binding fragment thereof comprises the sequence as set forth in SEQ ID NO: 16, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or more amino acids (e.g., a substitution, deletion, or addition of 1, 2, 3, 4, or 5 amino acids) as compared thereto. In some embodiments, the nanobody or antigen-binding fragment thereof comprises the sequence as set forth in SEQ ID NO: 16.

In some embodiments, the nanobody or antigen-binding fragment thereof comprises the sequence as set forth in SEQ ID NO: 18, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or more amino acids (e.g., a substitution, deletion, or addition of 1, 2, 3, 4, or 5 amino acids) as compared thereto. In some embodiments, the nanobody or antigen-binding fragment thereof comprises the sequence as set forth in SEQ ID NO: 18.

### Polypeptide construct

In another aspect, the present invention also provides a polypeptide construct capable of specifically binding to HER2, which comprises the nanobody or antigen-binding fragment thereof of the present invention, and an immunoglobulin Fc domain.

As used herein, the Fc domain is also referred to as the Fc region, which refers to a portion of the heavy chain constant region comprising CH2 and CH3. In some embodiments, the Fc domain comprises a hinge, CH2, and CH3. When the Fc domain contains a hinge, the hinge regulates dimerization between two Fc-containing polypeptides. The Fc domain can be any antibody heavy chain constant region isotype. In some embodiments, the Fc domain is IgG1, IgG2, IgG3, or IgG4.

In some embodiments, the Fc domain comprised in the polypeptide construct of the present invention is a natural Fc region, which comprises an amino acid sequence consistent with an amino acid sequence of Fc region found in nature. For example, the Fc domain can be the natural sequence human IgG1 Fc region, the natural sequence human IgG2 Fc region, the natural sequence human IgG3 Fc region, or the natural sequence human IgG4 Fc region. The natural Fc region may have effector functions. Exemplary "effector functions" include binding to Fc receptors; Clq binding and complement-dependent cytotoxicity (CDC); antibody-dependent cell-mediated cytotoxicity (ADCC); phage activity; downregulation of cell surface receptors (e.g., B cell receptors); and B cell activation, etc. Functional alterations can be achieved by replacing at least one amino acid residue in the natural Fc region with a different residue or by chemical modification. For example, altering the antibody's affinity for effector ligands (e.g., FcR or complement C1q) can change (e.g., reduce or enhance) effector functions.

Therefore, in some embodiments, the Fc domain comprised in the polypeptide construct of the present invention may also be a variant Fc region, which may comprise a mutation of one or more (e.g., 1 to 10, e.g., 1 to 5) amino acids or a chemical modification as compared to the natural Fc region to alter one or more of the following properties of the antibody of the present invention: Fc receptor binding, antibody glycosylation, number of cysteine residues, effector cell function, or complement function, etc. In some embodiments, the Fc domain comprised in the polypeptide construct of the present invention has a reduced or removed effector function, for example, it is an Fc domain that comprises a LALA mutation.

In some embodiments, the immunoglobulin Fc domain is optionally linked via a peptide linker to the N-terminus and/or C-terminus (e.g., C-terminus) of the nanobody or antigen-binding fragment thereof.

In some embodiments, the immunoglobulin Fc domain is an Fc domain of IgG (e.g., an Fc domain of IgG1, IgG2, IgG3, or IgG4).

In some embodiments, the immunoglobulin Fc domain is an Fc domain of human immunoglobulin, for example, an Fc domain of human IgG (e.g., an Fc domain of human IgG1, IgG2, IgG3, or IgG4).

In some embodiments, the immunoglobulin Fc domain comprises the sequence as set forth in SEQ ID NO: 19 or 20, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or more amino acids (e.g., a substitution, deletion, or addition of 1, 2, 3, 4, or 5 amino acids) as compared thereto. In some embodiments, the immunoglobulin Fc domain comprises the sequence as set forth in SEQ ID NO: 19 or 20.

In some embodiments, the polypeptide construct comprises the sequence as set forth in any one of SEQ ID NOs: 21-24, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or more amino acids (e.g., a substitution, deletion, or addition of 1, 2, 3, 4, or 5 amino acids) as compared thereto.

### Preparation of nanobody and polypeptide construct

The nanobody or polypeptide construct of the present invention can be prepared using various methods known in the art, such as by genetic engineering recombination techniques. For example, a DNA molecule encoding the nanobody or polypeptide construct of the present invention can be obtained by chemical synthesis or PCR amplification. The resulting DNA molecule is inserted into an expression vector and then transfected into a host cell. The transfected host cell is then cultured under specific conditions to express the antibody or polypeptide construct of the present invention.

In another aspect, the present invention also provides an isolated nucleic acid molecule, which encodes the nanobody or antigen-binding fragment thereof of the present invention, or the polypeptide construct of the present invention.

In another aspect, the present invention also provides a vector, which comprises the nucleic acid molecule of the present invention. In some embodiments, the vector is a cloning vector or an expression vector.

In another aspect, the present invention also provides a host cell, which comprises the nucleic acid molecule or vector of the present invention. Such host cell includes, but is not limited to, prokaryotic cell such as bacterial cell (e.g., *E. coli* cell), and eukaryotic cell such as fungal cell (e.g., yeast cell), insect cell, plant cell, and animal cell (e.g., mammalian cell, such as mouse cell, human cell, etc.).

In another aspect, the present invention also provides a method for preparing the nanobody or antigen-binding fragment thereof of the present invention or the polypeptide construct of the present invention, which comprises culturing the host cell of the present invention under a condition that allows protein expression, and recovering the nanobody or antigen-binding fragment thereof or the polypeptide construct from a culture of the cultured host cell.

### Bispecific or multispecific antibody

In another aspect, the present invention also provides a bispecific or multispecific antibody, which comprises the nanobody or antigen-binding fragment thereof of the present invention or the polypeptide construct of the present invention. The present invention also provides a use of the nanobody or antigen-binding fragment thereof or the polypeptide construct, or the nucleic acid molecule, vector or host cell encoding the same, as described in the present invention, in the manufacture of a bispecific or multispecific antibody.

In some embodiments, the bispecific or multispecific antibody is capable of specifically binding to HER2, and additionally specifically binding to one or more other targets.

In some embodiments, the bispecific or multispecific antibody further comprises at least one second antibody having a second binding specificity against a second target.

### Conjugate

In another aspect, the present invention also provides a conjugate, which comprises the nanobody or antigen-binding fragment thereof of the present invention, or the polypeptide construct of the present invention, or the bispecific or multispecific antibody of the present invention, and a conjugated portion connected thereto.

In some embodiments, the conjugated portion is selected from therapeutic agents (e.g., cytotoxic agent, cytokine, toxin, or radionuclide).

In some embodiments, the conjugate is an antibody-drug conjugate (ADC).

### Pharmaceutical composition

In another aspect, the present invention also provides a pharmaceutical composition, which comprises the nanobody or antigen-binding fragment thereof, the polypeptide construct, isolated nucleic acid molecule, vector, host cell, bispecific or multispecific antibody, or conjugate, as described in the present invention, and a pharmaceutically acceptable carrier and/or excipient.

In some embodiments, the pharmaceutical composition further comprises an additional pharmaceutically active agent, such as an antitumor agent.

In some embodiments, the nanobody or antigen-binding fragment thereof, polypeptide construct, isolated nucleic acid molecule, vector, host cell, bispecific or multispecific antibody, or conjugate, as described in the present invention, and the additional pharmaceutically active agent may be provided as separate components or as mixed components in the pharmaceutical composition.

The nanobody or antigen-binding fragment thereof, polypeptide construct, isolated nucleic acid molecule, vector, host cell, bispecific or multispecific antibody, conjugate, or pharmaceutical composition, as described in the present invention, can be formulated into any dosage form known in the medical field, such as tablet, pill, suspension, emulsion, solution, gel, capsule, powder, granule, elixir, lozenge, suppository, injection (including solution for injection, sterile powder for injection, and concentrated solution for injection), inhaler, spray, etc. Preferred dosage form depends on the intended route of administration and therapeutic use. A preferred dosage form is an injection. Such injections may be sterile injection solutions. For example, a sterile solution for injection can be prepared by incorporating the required dose of the antibody or antigen-binding fragment of the present invention into a suitable solvent, and optionally, simultaneously incorporating other desired components (including, but not limited to, pH adjuster, surfactant, adjuvant, ionic strength enhancer, isotonic agent, preservative, diluent, or any combination thereof), followed by sterile filtration. Alternatively, the sterile solution for injection can be prepared as a sterile lyophilized powder (e.g., by vacuum drying or freeze-drying) for easy storage and use. Such sterile lyophilized powders can be dispersed in a suitable carrier prior to use.

In some exemplary embodiments, the pharmaceutically acceptable carrier and/or excipient comprises a sterile injectable liquid (e.g., an aqueous or non-aqueous suspension or solution). In some exemplary embodiments, such sterile injectable liquid is selected from the group consisting of water for injection (WFI), bacteriostatic water for injection (BWFI), sodium chloride solution (e.g., 0.9% (w/v) NaCl), glucose solution (e.g., 5% glucose), surfactant-containing solution (e.g., 0.01% polysorbate 20), pH buffer solution (e.g., phosphate buffered solution), Ringer's solution, and any combination thereof.

The pharmaceutical composition of the present invention may comprise a "therapeutically effective amount" or a "prophylactically effective amount" of the nanobody or antigen-binding fragment thereof, isolated nucleic acid molecule, vector, host cell, bispecific or multispecific antibody, or conjugate, as described in the present invention. The "prophylactically effective amount" refers to an amount sufficient to prevent, arrest, or delay the onset of disease. The "therapeutically effective amount" refers to an amount sufficient to cure or at least partially arrest a disease and complications thereof in a patient already suffering from the disease. The therapeutically effective amount may vary depending on factors such as the severity of the disease to be treated, the overall state of the patient's own immune system, the patient's general characteristics such as age, weight, and sex, the method of drug administration, and other concurrent treatments, etc.

### Detection use

### Kit

In another aspect, the present invention also provides a kit, which comprises the nanobody or antigen-binding fragment thereof of the present invention or the polypeptide construct of the present invention.

In some embodiments, the kit comprises a conjugate, in which the conjugate comprises the nanobody or antigen-binding fragment thereof of the present invention or the polypeptide construct of the present invention, and a detectable label, such as an enzyme (e.g., horseradish peroxidase or alkaline phosphatase), a chemiluminescent reagent (e.g., acrid ester, luminol and derivative thereof, or ruthenium derivative), a fluorescent dye (e.g., fluorescein or fluorescent protein), a radionuclide, or biotin, linked to the nanobody or antigen-binding fragment thereof or polypeptide construct.

In some embodiments, the kit comprises the nanobody or antigen-binding fragment thereof of the present invention or the polypeptide construct of the present invention, and a second antibody that specifically recognizes the nanobody or antigen-binding fragment thereof or polypeptide construct; optionally, the second antibody further comprises a detectable label, such as an enzyme (e.g., horseradish peroxidase or alkaline phosphatase), a chemiluminescent reagent (e.g., acrid esters, luminol and derivative thereof, or ruthenium derivative), a fluorescent dye (e.g., fluorescein or fluorescent protein), a radionuclide, or biotin.

### Chimeric antigen receptor

In another aspect, the present application also provides a chimeric antigen receptor, which comprises an antigen-binding domain of the nanobody or antigen-binding fragment thereof as described above, or the polypeptide construct as described above.

In some embodiments, the antigen-binding domain is expressed by an immune effector cell (e.g., a T cell).

### Isolated nucleic acid molecule

In another aspect, the present application also provides an isolated nucleic acid molecule, which encodes the chimeric antigen receptor as described above.

### Vector

In another aspect, the present application also provides a vector, which comprises the isolated nucleic acid molecule as described above. In some embodiments, it is used to prepare a chimeric antigen receptor T cell.

### Host cell

In another aspect, the present application also provides a host cell, which comprises the isolated nucleic acid molecule as described above or the vector as described above. In some embodiments, the host cell is an immune effector cell (e.g., a T cell or NK cell). In some embodiments, the host cell is a chimeric antigen receptor T cell (CAR-T).

### Pharmaceutical use

In another aspect, the present invention also provides a use of the nanobody or antigen-binding fragment thereof, polypeptide construct, isolated nucleic acid molecule, vector, host cell, bispecific or multispecific antibody, conjugate, or pharmaceutical composition, as described in the present invention, in the manufacture of a medicament for prevention and/or treatment of a tumor in a subject.

In some embodiments, the tumor is an HER2-positive tumor.

In some embodiments, the medicament is used to inhibit the growth of an HER2-expressing tumor cell and/or kill the tumor cell.

In some embodiments, the medicament further comprises an additional pharmaceutically active agent.

In some embodiments, the additional pharmaceutically active agent is a drug with antitumor activity, such as an alkylating agent, mitotic inhibitor, antitumor antibiotic, antimetabolite, topoisomerase inhibitor, tyrosine kinase inhibitor, radionuclide agent, radiosensitizer, antiangiogenic agent, cytokine, molecularly targeted drug, immune checkpoint inhibitor, or oncolytic virus.

In some embodiments, the tumor is selected from the group consisting of solid tumors, such as gastric cancer, lung cancer, liver cancer, head and neck cancer, skin cancer, colorectal cancer, ovarian cancer, esophageal cancer, pancreatic cancer, cervical cancer, mesothelioma, breast cancer, colorectal cancer, or oral squamous cell carcinoma.

In some embodiments, the subject is a mammal, such as a human.

### Method for prevention and/or treatment of tumor

In another aspect, the present invention also provides a method for preventing and/or treating of a tumor in a subject, comprising: administering to the subject in need an effective amount of the nanobody or antigen-binding fragment thereof, polypeptide construct, isolated nucleic acid molecule, vector, host cell, bispecific or multispecific antibody, conjugate, or pharmaceutical composition, as described in the present invention.

In some embodiments, the tumor is an HER2-positive tumor.

In some embodiments, the tumor is selected from the group consisting of solid tumors, such as gastric cancer, lung cancer (e.g., non-small cell lung cancer), liver cancer, head and neck cancer, skin cancer, colorectal cancer, ovarian cancer, esophageal cancer, pancreatic cancer, cervical cancer, mesothelioma, breast cancer, colorectal cancer, or oral squamous cell carcinoma.

In some embodiments, the subject is a mammal, such as a human.

The nanobody or antigen-binding fragment thereof, polypeptide construct, bispecific or multispecific antibody, conjugate, or pharmaceutical composition, as described in the present invention, can be formulated into any dosage form known in the medical field, such as tablet, pill, suspension, emulsion, solution, gel, capsule, powder, granule, elixir, lozenge, suppository, injection (including solution for injection, sterile powder for injection, and concentrated solution for injection), inhaler, spray, etc. Preferred dosage form depends on the intended route of administration and therapeutic use. The nanobody or antigen-binding fragment thereof, polypeptide construct, bispecific or multispecific antibody, conjugate, or pharmaceutical composition, as described in the present invention, should be sterile and stable under manufacturing and storage conditions. A preferred dosage form is an injection. Such injection may be a sterile solution for injection. For example, a sterile solution for injection can be prepared by incorporating an appropriate dose of the nanobody or antigen-binding fragment, polypeptide construct, bispecific or multispecific antibody, conjugate, or pharmaceutical composition, as described in the present invention, and optionally, simultaneously incorporating other desired components (including, but not limited to, pH adjuster, surfactant, adjuvant, ionic strength enhancer, isotonic agent, preservative, diluent, or any combination thereof) into a suitable solvent, followed by sterile filtration. Alternatively, a sterile solution for injection can be prepared as a sterile lyophilized powder (e.g., by vacuum drying or freeze-drying) for easy storage and use. Such sterile lyophilized powder can be dispersed in a suitable carrier before use, such as water for injection (WFI), antibacterial water for injection (BWFI), sodium chloride solution (e.g., 0.9% (w/v) NaCl), glucose solution (e.g., 5% glucose), surfactant-containing solution (e.g., 0.01% polysorbate 20), pH buffer solution (e.g., phosphate buffered solution), Ringer's solution, and any combination thereof.

The nanobody or antigen-binding fragment thereof, polypeptide construct, bispecific or multispecific antibody, conjugate, or pharmaceutical composition, as described in the present invention can be administered by any suitable method known in the art, including but not limited to oral, buccal, sublingual, ocular, topical, parenteral, rectal, intrathecal, intracisternal (within cisterna of endoplasmic reticulum), groin, intrabladder, topical (e.g., powder, ointment, or drop), or nasal routes. However, for many therapeutic applications, the preferred route/mode of administration is parenteral administration (e.g., intravenous injection or bolus, subcutaneous injection, intraperitoneal injection, intramuscular injection). Those skilled in the art will understand that the route and/or mode of administration will vary depending on the intended purpose. In certain embodiments, the nanobody or antigen-binding fragment thereof, polypeptide construct, bispecific or multispecific antibody, conjugate, or pharmaceutical composition of the present invention is administered by intravenous injection or bolus injection.

### Detection method

In another aspect, the present invention also provides a method for detecting the presence or amount of HER2 in a sample, which comprises using the nanobody or antigen-binding fragment thereof, or polypeptide construct of the present invention. In some embodiments, the method is used for therapeutic purposes, diagnostic purposes, or non-therapeutic and non-diagnostic purposes.

In some embodiments, the method is an immunological assay, such as Western blotting, enzyme immunoassay (e.g., ELISA), chemiluminescent immunoassay, fluorescence immunoassay, or radioimmunoassay.

In some embodiments, the method comprises using a conjugate, wherein the conjugate comprises the nanobody or antigen-binding fragment thereof of the present invention or the polypeptide construct of the present invention, and a detectable label, such as an enzyme (e.g., horseradish peroxidase or alkaline phosphatase), a chemiluminescent reagent (e.g., acrid esters, luminol and derivative thereof, or ruthenium derivative), a fluorescent dye (e.g., fluorescein or fluorescent protein), a radionuclide, or biotin, that is linked to the nanobody or antigen-binding fragment thereof or polypeptide construct.

In some embodiments, the method comprises using the nanobody or antigen-binding fragment thereof of the present invention or the polypeptide construct of the present invention, and the method further comprises using a second antibody bearing a detectable label (e.g., an enzyme (e.g., horseradish peroxidase or alkaline phosphatase), a chemiluminescent reagent (e.g., acrid esters, luminol and derivative thereof, or ruthenium derivative), a fluorescent dye (e.g., fluorescein or fluorescent protein), a radionuclide, or biotin) to detect the nanobody or antigen-binding fragment thereof or polypeptide construct.

In some embodiments, the method comprises: (1) contacting the sample with the nanobody or antigen-binding fragment thereof of the present invention, the polypeptide construct of the present invention, or a conjugate, wherein the conjugate comprises the nanobody or antigen-binding fragment thereof of the present invention, or the polypeptide construct of the present invention, and a detectable label, such as an enzyme (e.g., horseradish peroxidase or alkaline phosphatase), a chemiluminescent reagent (e.g., acrid esters, luminol and derivative thereof, or ruthenium derivative), a fluorescent dye (e.g., fluorescein or fluorescent protein), a radionuclide, or biotin, that is linked to the nanobody or antigen-binding fragment thereof or polypeptide construct; and (2) detecting the formation of an antigen-antibody immune complex or detecting the amount of the immune complex. The formation of the immune complex indicates the presence of HER2 or cells expressing HER2.

In some embodiments, the method is used to detect whether a tumor can be treated by an HER2-targeted antitumor therapy. In such embodiments, the sample is from a subject who has a tumor, is suspected of having a tumor, or is at risk of having a tumor. In some embodiments, the sample is a cell sample (e.g., a sample containing tumor cell) or a body fluid sample (e.g., blood) from a subject (e.g., a mammal, such as a human). In some embodiments, the presence of HER2 or HER2-expressing cells, and/or an increase in the amount of HER2 or HER2-expressing cells compared to a reference level (e.g., compared to a patient without tumor disease), indicates that the subject is suitable for an HER2-targeted antitumor therapy.

### Use in manufacture of detection reagent

In another aspect, the present invention also provides a use of the nanobody or antigen-binding fragment thereof of the present invention or the polypeptide construct of the present invention in the manufacture of a detection reagent for detecting the presence or amount of HER2 in a sample or for detecting whether a tumor can be treated by an HER2-targeted antitumor therapy.

In some embodiments, the detection reagent detects the presence or amount of HER2 in a sample and optionally detects whether a tumor can be treated by an HER2-targeted antitumor therapy by the method of the present invention as described above.

In some embodiments, the sample is a cell sample (e.g., a sample containing tumor cells) or a body fluid sample (e.g., blood) from a subject (e.g., a mammal, such as a human).

### Definitions of terms

In the present invention, unless otherwise stated, scientific and technical terms used herein have the meanings commonly understood by those skilled in the art. Furthermore, the virological, biochemical, and immunological laboratory procedures used herein are standard procedures widely used in the respective fields. Meanwhile, for a better understanding of the present invention, definitions and explanations of relevant terms are provided below.

When the terms "e.g.," "such as," "comprising," "including," "containing," or variations thereof are used herein, these terms shall not be considered limiting terms but shall be construed as meaning "but not limited to" or "not limited to."

Unless otherwise specified herein or clearly contradicted by the context, the terms "an," "a," "the," and similar designations shall be construed to cover both the singular and plural in the context of describing the present invention (particularly in the context of the following claims).

As used herein, the term "camelid antibody" refers to an antibody against an antigen produced by an immunized or antigen-invaded *Camelidae* animal (including *Camel*, *Alpaca*, and *L. glama*). It is known to those skilled in the art that antibodies produced by *Camelidae* animals include "heavy-chain antibodies" (Camelid heavy-chain antibody, HCAb) that lack light chains. These antibodies consist solely of a variable domain of the heavy chain of HCAb (VHH) and two conventional CH2 and CH3 regions. The VHH domain, when cloned and expressed independently, exhibits excellent structural stability and antigen-binding activity. VHH is currently the smallest known unit capable of binding to a target antigen.

As used herein, the terms "nanobody" or "single-domain antibody" have the meanings commonly understood by those skilled in the art and are used interchangeably. They refer to antibody fragments composed of a single monomeric variable antibody domain (e.g., a single heavy chain variable region), typically derived from the variable region of heavy chain antibodies (e.g., camelid antibodies or shark antibodies). Typically, a nanobody consists of four framework regions and three complementarity-determining regions, with a structure of FR1-CDR1-FR2-CDR2-FR3-CDR3-FR4. Nanobodies may be truncated at the N-terminus or C-terminus to include only part of FR1 and/or FR4, or to lack one or both of those framework regions, as long as they substantially retain antigen binding ability and specificity.

As used herein, the term "antigen-binding fragment" of nanobody refers to a polypeptide comprising a fragment of nanobody that retains the ability to specifically bind to the same antigen bound by the nanobody, and/or competes with the nanobody for specific binding to the antigen, which is also referred to as an "antigen-binding fragment." See generally Fundamental Immunology, Ch. 7 (Paul, W., ed., 2nd ed., Raven Press, N.Y. (1989), which is incorporated herein by reference in its entirety for all purposes. Antigen-binding fragments of the nanobody of the present invention can be produced by recombinant DNA techniques or by enzymatic or chemical cleavage of the nanobody of the present invention. In some embodiments, the "antigen-binding fragment" of nanobody may be truncated at the N-terminus or C-terminus compared to the full-length nanobody so as to comprise only a portion of FR1 and/or FR4, or lack one or both of those framework regions, as long as it substantially retains antigen binding ability and specificity.

Antigen-binding fragments of nanobodies can be obtained from a given nanobody (e.g., the nanobody provided by the present invention) using conventional techniques known to those skilled in the art (e.g., recombinant DNA technology or enzymatic or chemical cleavage), and the antigen-binding fragments of the nanobody can be screened for specificity in the same manner as for the complete nanobody.

As used herein, unless the context explicitly states otherwise, the terms "single-domain antibody" or "nanobody" refer not only to the complete nanobody but also to the antigen-binding fragment of the nanobody.

As used herein, the term "complementarity-determining region" or "CDR" refers to the amino acid residues in the variable region of an antibody responsible for antigen binding. Nanobodies contain three CDRs, designated CDR1, CDR2, and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al., Dev). The CDRs of nanobodies are defined in Comparat. Immunol. 27:55-77, 2003. For a given nanobody, those skilled in the art will readily identify the CDRs as defined by various numbering systems. Furthermore, the correspondences between different numbering systems are well known to those skilled in the art (e.g., see Lefranc et al., Dev. Comparat. Immunol. 27:55-77, 2003). As used herein, the CDRs of nanobodies are preferably determined using the Kabat, Chothia, and/or IMGT numbering systems.

As used herein, the term "framework region" or "FR" residues refer to those amino acid residues in a variable region of the antibody other than the CDR residues defined above.

As used herein, the term "Fc domain" or "Fc region" refers to a fragment of the heavy chain constant region comprising CH2 and CH3. The Fc fragment of an antibody has a variety of different functions but does not participate in antigen binding. "Effector functions" mediated by the Fc region include Fc receptor binding; Clq binding and complement-dependent cytotoxicity (CDC); antibody-dependent cell-mediated cytotoxicity (ADCC); phagocytosis; down-regulation of cell surface receptors (e.g., B cell receptor); and B cell activation, among others. In some embodiments, the Fc region comprises a hinge, CH2, and CH3. When the Fc region comprises a hinge, the hinge regulates dimerization between two Fc-containing polypeptides. The Fc region can be of any antibody heavy chain constant region isotype, such as IgG1, IgG2, IgG3, or IgG4.

The Fc domain may comprise either a native Fc region or a variant Fc region. A native Fc region comprises an amino acid sequence that is consistent with the amino acid sequence of an Fc region found in nature. For example, native sequence human Fc regions include native sequence human IgG1 Fc region (non-A and A allotypes); native sequence human IgG2 Fc region; native sequence human IgG3 Fc region; and native sequence human IgG4 Fc region, as well as naturally occurring variants thereof. A variant Fc region comprises an amino acid sequence that differs from the amino acid sequence of a native Fc region due to at least one amino acid modification. In some embodiments, the variant Fc region may exhibit altered effector functions compared to the native Fc region (e.g., Fc receptor binding, antibody glycosylation, the number of cysteine residues, effector cell function, or complement function).

As used herein, the term "humanized antibody" refers to a genetically engineered non-human antibody whose amino acid sequence has been modified to increase its homology with the sequence of a human antibody. Generally, all or part of the CDR regions of a humanized antibody are derived from a non-human antibody (donor antibody), while all or part of the non-CDR regions (e.g., framework regions (FR) of variable region and/or constant region) are derived from a human immunoglobulin (acceptor antibody). In certain embodiments, the CDR regions of a humanized antibody are derived from a non-human antibody (donor antibody), and all or part of the non-CDR regions (e.g., variable region FR and/or constant region) are derived from a human immunoglobulin (acceptor antibody). Humanized antibodies typically retain the desired properties of the donor antibody, including but not limited to antigen specificity, affinity, reactivity, and the like. In the present application, the donor antibody may be a camelid antibody with desired properties (e.g., antigen specificity, affinity, reactivity, etc.). To prepare humanized antibodies, the CDR regions of the immunized animal can be inserted into human framework sequences using methods known in the art. In the context of nanobodies, a humanized antibody may refer to a humanized VHH, i.e., a VHH in which one or more framework regions have been substantially replaced with human framework regions. In some cases, certain framework regions (FR) of human immunoglobulin may be substituted with corresponding non-human residues. Additionally, humanized VHH may contain residues not found in the original VHH or human framework sequences but included to further improve and optimize the performance of the VHH or VHH-containing polypeptides.

As used herein, the term "identity" refers to the degree of sequence matching between two polypeptides or between two nucleic acids. To determine the percent identity between two amino acid sequences or two nucleic acid sequences, the sequences are aligned for optimal comparison purposes (e.g., gaps may be introduced in the first amino acid sequence or nucleic acid sequence to achieve optimal alignment with the second amino acid or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, the molecules are identical at that position. The percent identity between the two sequences is a function of the number of identical positions shared by the sequences (i.e., percent identity = number of identical overlapping positions / total number of positions × 100%). In certain embodiments, the two sequences are of the same length.

Determination of percent identity between two sequences can also be accomplished using a mathematical algorithm. A non-limiting example of a mathematical algorithm utilized for the comparison of two sequences is the algorithm of Karlin and Altschul, 1990, Proc. Natl. Acad. Sci. U.S.A. 87:2264-2268, as modified in Karlin and Altschul, 1993, Proc. Natl. Acad. Sci. U.S.A. 90:5873-5877. Such an algorithm is integrated into the NBLAST and XBLAST programs of Altschul et al., 1990, J. Mol. Biol. 215:403.

As used herein, the term "specific binding" refers to a non-random binding reaction between two molecules, such as the reaction between an antibody and its target antigen. The strength or affinity of a specific binding interaction can be expressed in terms of the equilibrium dissociation constant (K_{D}) of the interaction. In the present invention, the term "K_{D}" refers to the equilibrium dissociation constant of a particular antibody-antigen interaction, which is used to describe the binding affinity between the antibody and the antigen. The smaller the equilibrium dissociation constant, the tighter the antibody-antigen binding and the higher the affinity between the antibody and the antigen.

The specific binding properties between two molecules can be determined using methods well known in the art. One method involves measuring the rates of formation and dissociation of antigen-binding site/antigen complexes. Both the "association rate constant" (ka or kon) and the "dissociation rate constant" (kdis or koff) can be calculated based on concentration and the actual rates of association and dissociation (see Malmqvist M, Nature, 1993, 361:186-187). The ratio of kdis/kon equals the dissociation constant K_{D} (see Davies et al., Annual Rev Biochem, 1990; 59:439-473). The values of K_{D}, kon, and kdis can be measured using any effective method. In some embodiments, surface plasmon resonance (SPR) can be used to measure the dissociation constant in Biacore. Additionally, bio-layer interferometry or Kinexa can be used to measure the dissociation constant.

As used herein, the detectable label of the present invention can be any substance detectable by fluorescent, spectroscopic, photochemical, biochemical, immunological, electrical, optical, or chemical means. Such labels are well known in the art, and examples thereof include, but are not limited to, enzymes (e.g., horseradish peroxidase, alkaline phosphatase, β-galactosidase, urease, glucose oxidase, etc.), radionuclides (e.g., ³H, ¹²⁵I, ³⁵S, ¹⁴C, or ³²P), fluorescent dyes (e.g., fluorescein isothiocyanate (FITC), fluorescein, tetramethylrhodamine isothiocyanate (TRITC), phycoerythrin (PE), Texas Red, rhodamine, quantum dots, or cyanine dye derivatives such as Cy7, Alexa 750), luminescent substances (e.g., chemiluminescent substances such as acridinium esters, luminol and derivatives thereof, ruthenium derivatives such as tris(bipyridine)ruthenium), magnetic beads (e.g., Dynabeads^{®}), calorimetric labels such as colloidal gold, or colored glass or plastic (e.g., polystyrene, polypropylene, latex, etc.) beads, and biotin for binding with avidin (e.g., streptavidin) modified with the aforementioned labels.

As used herein, the term "vector" refers to a nucleic acid delivery vehicle into which a polynucleotide can be inserted. When a vector enables the expression of the protein encoded by the inserted polynucleotide, it is referred to as an expression vector. A vector can be introduced into a host cell by transformation, transduction, or transfection, allowing the genetic material elements it carries to be expressed in the host cell. Vectors are well known to those skilled in the art and include, but are not limited to: plasmids; phagemids; cosmids; artificial chromosomes such as yeast artificial chromosome (YAC), bacterial artificial chromosome (BAC), or P1-derived artificial chromosome (PAC); bacteriophages such as λ phage or M13 phage; and animal viruses. Animal viruses that can be used as vectors include, but are not limited to, retroviruses (including lentivirus), adenoviruses, adeno-associated viruses, herpes viruses (e.g., herpes simplex virus), poxviruses, baculoviruses, papillomaviruses, and papovaviruses (e.g., SV40). A vector may contain multiple elements that control expression, including but not limited to promoter sequence, transcription initiation sequence, enhancer sequence, selection element, and reporter gene. Additionally, a vector may contain an origin of replication.

As used herein, the term "host cell" refers to a cell into which a vector can be introduced, including but not limited to, prokaryotic cells such as *Escherichia coli* or *Bacillus subtilis*, fungal cells such as yeast cells or *Aspergillus*, insect cells such as *S2 Drosophila* cells or *Sf9* cells, or animal cells such as fibroblasts, CHO cells, COS cells, NSO cells, HeLa cells, BHK cells, HEK 293 cells, or human cells.

As used herein, the term "prevention" refers to a method implemented to prevent or delay the occurrence of a disease or disorder or symptom (e.g., tumor) in a subject. As used herein, the term "treatment" refers to a method implemented to obtain a beneficial or desired clinical outcome. For the purposes of the present invention, the beneficial or desired clinical outcome includes, but is not limited to, alleviation of symptom, reduction of the extent of the disease, stabilization (i.e., not worsening) of the state of the disease, delay or slowing of the progression of the disease, amelioration or palliation of the state of the disease, and remission of symptom (whether partial or total), whether detectable or undetectable. In addition, "treatment" can also refer to prolonged survival compared to expected survival (if not receiving treatment).

As used herein, the terms "cancer" or "tumor" are used interchangeably and refer to a broad class of diseases characterized by the uncontrolled growth of abnormal cells in the body. Uncontrolled cell division can lead to the formation of malignant tumors or cells that invade adjacent tissues and may metastasize to distant sites of the body via the lymphatic system or bloodstream. Cancer includes benign and malignant cancers, as well as dormant tumors or micrometastases. Cancer also includes hematologic malignancies.

### Beneficial effects of the invention

The present application provides an anti- HER2 nanobody or polypeptide construct, which exhibits high binding activity to HER2. In particular, the nanobody or polypeptide construct of the invention possesses internalization activity and can mediate the entry of toxins into cells through internalization, thereby exerting a killing effect on tumor cells. On this basis, humanized nanobodies or polypeptide constructs prepared from the aforementioned nanobody or polypeptide construct still retain these outstanding activities. Moreover, the nanobody or polypeptide construct also offers advantages such as small molecular weight and ease of production.

The embodiments of the present invention will be described in detail below with reference to the accompanying drawings and examples. However, those skilled in the art will understand that the following drawings and examples are for illustrative purposes only and are not intended to limit the scope of the invention. Various objects and advantages of the present invention will become apparent to those skilled in the art from the following detailed description of the drawings and preferred embodiments.

### Brief Description of the Drawings

Figure 1 shows the binding activities of the candidate antibody to the human HER2 antigen detected by ELISA.
Figure 2 shows the binding activities of the candidate antibody to SK-BR-3 cells in a FACS experiment.
Figure 3A shows the internalization percentage of the candidate antibody in SK-BR-3 cells.
Figure 3B shows the MFI reduction of the candidate antibody on the surface of SK-BR-3 cells.
Figure 4 shows the cytotoxic activities of the candidate antibody-Fab-ZAP on SK-BR-3 cells.
Figure 5A shows the test results for whether the candidate antibody competitively binds to recombinant human HER2 with Trastuzumab.
Figure 5B shows the test results for whether the candidate antibody competitively binds to recombinant human HER2 with Pertuzumab.
Figure 6 shows the ELISA binding curves of the MHAB14-10 humanized antibodies to recombinant human HER2.
Figure 7 shows the ELISA binding curves of the MHAB14-14 humanized antibodies to recombinant human HER2.
Figure 8 shows the flow cytometry binding curves of the MHAB14-10 humanized antibodies to SK-BR-3 cells.
Figure 9 shows the flow cytometry binding curves of the MHAB14-14 humanized antibodies to SK-BR-3 cells.
Figure 10 shows the percentages of internalization of the humanized antibodies in SK-BR-3 cells.
Figure 11 shows the killing curve of the humanized antibody-Fab-ZAP in SK-BR-3 cells.
Figure 12 shows the test results for whether the antibody MHAB14-14 competitively binds to the cell surface HER2 antigen with trastuzumab or pertuzumab.
Figure 13 shows the ELISA binding curves of the antibody MHAB14-14-6 to HER2 from different species.
Figure 14 shows the ELISA binding curves of the antibody MHAB14-14-6 to EGFR family proteins.

### Sequence information

Information on some sequences involved in the present invention is provided in Table 1 below.

**Table 1: Description of sequences**

| SEQ ID NO: | Sequence name | Sequence |
|---|---|---|
| 1 | MHAB14-10-Kabat-CDR1/ MHAB14-10-5-Kabat-CDR1 | HYTMS |
| 2 | MHAB14-10-Kabat-CDR2 | SITYGGVTTNYADSVKG |
| 3 | MHAB14-10-5-Kabat-CDR2 | SITYGGVTTYYADSVKG |
| 4 | MHAB14-10-Kabat-CDR3/ MHAB14-10-5-Kabat-CDR3 | DWTAAGLNH |
| 5 | MHAB14-10-IMGT-CDR1/ MHAB14-10-5-IMGT-CDR1 | GITFRHYT |
| 6 | MHAB14-10-IMGT-CDR2/ MHAB14-10-5-IMGT-CDR2 | ITYGGVTT |
| 7 | MHAB14-10-IMGT-CDR3/ MHAB14-10-5-IMGT-CDR3 | HADWTAAGLNH |
| 8 | MHAB14-14-Kabat-CDR1/ MHAB14-14-6-Kabat-CDR1 | NYPMN |
| 9 | MHAB14-14-Kabat-CDR2 | VITRDGGVTRYAASVKG |
| 10 | MHAB14-14-6-Kabat-CDR2 | VITRDGGVTYYADSVKG |
| 11 | MHAB14-14-Kabat-CDR3/ MHAB14-14-6-Kabat-CDR3 | PNYNA |
| 12 | MHAB14-14-IMGT-CDR1/ MHAB14-14-6-IMGT-CDR1 | GFIFGNYP |
| 13 | MHAB14-14-IMGT-CDR2/ MHAB14-14-6-IMGT-CDR2 | ITRDGGVT |
| 14 | MHAB14-14-IMGT-CDR3/MHAB14-14-6-IMGT-CDR3 | HNPNYNA |
| 15 | MHAB14-10 VHH | QVQLVESGGGMVQPGGSLRLSCAASGITFRHYTMSW YRQAPGKERELVASITYGGVTTNYADSVKGRFTLSR DDAKTTAYLQMTGLKPEDTAVYYCHADWTAAGLN HWGQGTQVTVSS |
| 16 | MHAB14-10-5 VHH | QVQLVESGGGLVQPGGSLRLSCAASGITFRHYTMSW VRQAPGKERELVASITYGGVTTYYADSVKGRFTISRD NAKNTLYLQMNSLRPEDTAVYYCHADWTAAGLNH WGQGTQVTVSS |
| 17 | MHAB14-14 VHH | QVQLVESGGGLVQPGGSLRLSCAASGFIFGNYPMNW YRQAPGKEREFVAVITRDGGVTRYAASVKGRFTITR DNAKNTVFLQMNSLKLEDTAVYYCHNPNYNAWGQ GTQVTVAS |
| 18 | MHAB14-14-6 VHH | QVQLVESGGGLVQPGGSLRLSCAASGFIFGNYPMNW YRQAPGKEREFVAVITRDGGVTYYADSVKGRFTISR DNAKNTVYLQMNSLRAEDTAVYYCHNPNYNAWGQ GTQVTVSS |
| 19 | Fc-1 | EPKSSDKTHTCPPCPAPEAAGGPSVFLFPPKPKDTLMI SRTPEVTCVVVDVSHEDPEVKFNWYVDGVEVHNAK TKPREEQYNSTYRVVSVLTVLHQDWLNGKEYKCKV SNKALPAPIEKTISKAKGQPREPQVYTLPPSREEMTK NQVSLTCLVKGFYPSDIAVEWESNGQPENNYKTTPP VLDSDGSFFLYSKLTVDKSRWQQGNVFSCSVMHEAL HNHYTQKSLSLSPGK |
| 20 | Fc-2 | EPKSCDKTHTCPPCPAPEAAGGPSVFLFPPKPKDTLMI SRTPEVTCVVVDVSHEDPEVKFNWYVDGVEVHNAK TKPREEQYNSTYRVVSVLTVLHQDWLNGKEYKCKV SNKALPAPIEKTISKAKGQPREPQVYTLPPSREEMTK NQVSLTCLVKGFYPSDIAVEWESNGQPENNYKTTPP VLDSDGSFFLYSKLTVDKSRWQQGNVFSCSVMHEAL HNHYTQKSLSLSPGK |
| 21 | MHAB14-10 | QVQLVESGGGMVQPGGSLRLSCAASGITFRHYTMSW YRQAPGKERELVASITYGGVTTNYADSVKGRFTLSR DDAKTTAYLQMTGLKPEDTAVYYCHADWTAAGLN HWGQGTQVTVSSEPKSSDKTHTCPPCPAPEAAGGPS VFLFPPKPKDTLMISRTPEVTCVVVDVSHEDPEVKFN WYVDGVEVHNAKTKPREEQYNSTYRVVSVLTVLHQ DWLNGKEYKCKVSNKALPAPIEKTISKAKGQPREPQ VYTLPPSREEMTKNQVSLTCLVKGFYPSDIAVEWESN GQPENNYKTTPPVLDSDGSFFLYSKLTVDKSRWQQG NVFSCSVMHEALHNHYTQKSLSLSPGK |
| 22 | MHAB14-10-5 | QVQLVESGGGLVQPGGSLRLSCAASGITFRHYTMSW VRQAPGKERELVASITYGGVTTYYADSVKGRFTISRD NAKNTLYLQMNSLRPEDTAVYYCHADWTAAGLNH WGQGTQVTVSSEPKSCDKTHTCPPCPAPEAAGGPSV FLFPPKPKDTLMISRTPEVTCVVVDVSHEDPEVKFNW YVDGVEVHNAKTKPREEQYNSTYRVVSVLTVLHQD WLNGKEYKCKVSNKALPAPIEKTISKAKGQPREPQV YTLPPSREEMTKNQVSLTCLVKGFYPSDIAVEWESNG QPENNYKTTPPVLDSDGSFFLYSKLTVDKSRWQQGN VFSCSVMHEALHNHYTQKSLSLSPGK |
| 23 | MHAB14-14 | QVQLVESGGGLVQPGGSLRLSCAASGFIFGNYPMNW YRQAPGKEREFVAVITRDGGVTRYAASVKGRFTITR DNAKNTVFLQMNSLKLEDTAVYYCHNPNYNAWGQ GTQVTVASEPKSSDKTHTCPPCPAPEAAGGPSVFLFP PKPKDTLMISRTPEVTCVVVDVSHEDPEVKFNWYVD GVEVHNAKTKPREEQYNSTYRVVSVLTVLHQDWLN GKEYKCKVSNKALPAPIEKTISKAKGQPREPQVYTLP PSREEMTKNQVSLTCLVKGFYPSDIAVEWESNGQPE NNYKTTPPVLDSDGSFFLYSKLTVDKSRWQQGNVFS CSVMHEALHNHYTQKSLSLSPGK |
| 24 | MHAB14-14-6 | QVQLVESGGGLVQPGGSLRLSCAASGFIFGNYPMNW YRQAPGKEREFVAVITRDGGVTYYADSVKGRFTISR DNAKNTVYLQMNSLRAEDTAVYYCHNPNYNAWGQ GTQVTVSSEPKSCDKTHTCPPCPAPEAAGGPSVFLFPP KPKDTLMISRTPEVTCVVVDVSHEDPEVKFNWYVDG VEVHNAKTKPREEQYNSTYRVVSVLTVLHQDWLNG KEYKCKVSNKALPAPIEKTISKAKGQPREPQVYTLPP SREEMTKNQVSLTCLVKGFYPSDIAVEWESNGQPEN NYKTTPPVLDSDGSFFLYSKLTVDKSRWQQGNVFSC SVMHEALHNHYTQKSLSLSPGK |

### Specific Models for Carrying Out the present invention

The present invention will now be described with reference to the following embodiments, which are intended to illustrate the present invention (but not limit it).

Unless otherwise specified, the molecular biology experimental methods and immunoassays used in the present invention are substantially in accordance with the methods described in J. Sambrook et al., Molecular Cloning: A Laboratory Manual, 2nd Edition, Cold Spring Harbor Laboratory Press, 1989, and F. M. Ausubel et al., A Concise Guide to Molecular Biology, 3rd Edition, John Wiley & Sons, Inc., 1995; the use of restriction endonucleases is in accordance with the manufacturer's recommendations. Those skilled in the art will understand that the examples illustrate the present invention by way of example and are not intended to limit the scope sought to be protected by the present invention.

### Example 1. Obtaining the anti-human HER2 nanobody sequence

### 1-1 Alpaca immunization and serum titer detection

A blank alpaca was immunized with the Her2/ERBB2 Protein, Human, Recombinant (ECD, His Tag) antigen (Sinobiological, Cat. No. 10004-H08H). 10 mL of blood was collected before the initial immunization to serve as negative serum. The initial immunization used a 1:1 mixture of complete Freund's adjuvant and 1 mg of HER2 antigen, administered subcutaneously at multiple sites. Subsequently, the second, third, fourth, fifth, and sixth immunizations were performed at weeks 3, 5, 7, 9 and 11 using a 1:1 mixture of incomplete Freund's adjuvant and 0.5 mg of HER2 antigen, administered subcutaneously at multiple sites. 10 mL of peripheral blood was collected before each of the third, fourth, fifth, and sixth immunizations, and serum was separated. Immune responses were monitored using ELISA. Five days after the sixth immunization, 50 mL of peripheral blood was collected for subsequent nanobody library construction.

### 1-2 Screening and identification of specific nanobodies

Peripheral blood was collected from immunized alpacas, RNA was extracted, and cDNA samples were prepared. The VHH antibody-encoding gene was cloned by PCR, and a single-domain antibody phage display library was constructed. Clones were randomly selected, and the library size and diversity were identified.

Using the Her2/ERBB2 Protein, Human, Recombinant (ECD, His Tag) antigen (Sinobiological, Cat. No. 10004-H08H), the single-domain antibody phage display library was enriched through panning. Single clones were then selected for Phage-ELISA detection to identify candidate clones with different amino acid sequences in the CDR region. Finally, 40 unique sequences were selected.

### 1-3 Unique sequence solubility verification and antibody expression

The 40 positive clone plasmids were extracted and individually transformed into *E. coli Rosetta.* Expression was induced overnight with 0.4 mM IPTG at 20 °C with shaking at 200 rpm. The next day, the cultures were centrifuged, and the pellets were resuspended in PBS, followed by ultrasonication for disruption. After centrifugation, the lysate supernatants from the 40 clones were collected. The binding activity of the lysate supernatants from the 40 clones to the human HER2 antigen was assessed by ELISA (OD₄₅₀ values are shown in Table 2).

Twenty-six unique sequences with strong binding activity to human HER2 were selected. The VHH sequences were fused to the Fc (LALA) sequence (SEQ ID NO: 19) of human IgG1 and transiently expressed in CHO cells to produce the MHAB14 candidate antibodies (the correspondence between the numbers in the present application and the selected unique sequences is shown in Table 3; in the following description, the MHAB14 candidate antibodies are referred to by their numbers in the present application, i.e., MHAB14-1 to MHAB14-26). After expression, the antibodies were purified using Protein A, and dissolved in PBS. The purity of the antibody solutions (SEC, 280 nm) was >95%, and endotoxin levels were <1 EU/mg. The antibodies were aliquoted and stored at -80 °C.

**Table 2. OD₄₅₀ results for the 40 clones**

| Unique sequence | OD₄₅₀ | Unique sequence | OD₄₅₀ |
|---|---|---|---|
| M26H2-2001 | 2.616 | M26H2-3013 | 0.787 |
| M26H2-2002 | 0.804 | M26H2-3015 | 1.234 |
| M26H2-2030 | 2.077 | M26H2-3016 | 1.847 |
| M26H2-2032 | 0.809 | M26H2-3018 | 0.081 |
| M26H2-2043 | 1.5 | M26H2-3027 | 0.863 |
| M26H2-2048 | 1.759 | M26H2-3038 | 1.014 |
| M26H2-2073 | 1.241 | M26H2-3044 | 0.085 |
| M26H2-2079 | 1.988 | M26H2-3050 | 2.504 |
| M26H2-2081 | 1.716 | M26H2-3052 | 0.397 (Weak Positive) |
| M26H2-2087 | 1.296 | M26H2-3055 | 0.115 |
| M26H2-2089 | 1.341 | M26H2-3056 | 2.191 |
| M26H2-2109 | 1.57 | M26H2-3076 | 0.196 |
| M26H2-2136 | 0.243 (Weak Positive) | M26H2-3088 | 1.624 |
| M26H2-2141 | 0.69 | M26H2-3094 | 1.903 |
| M26H2-2150 | 1.475 | M26H2-3107 | 0.133 |
| M26H2-2152 | 0.851 | M26H2-3134 | 1.03 |
| M26H2-2168 | 2.261 | M26H2-3147 | 1.068 |
| M26H2-2179-4 | 1.314 | M26H2-3168 | 0.087 |
| M26H2-3011 | 2.614 | M26H2-3176 | 1.035 |
| M26H2-3012 | 2.172 | M26H2-3186 | 0.131 |
| Irrelevant | 0.073 | 1%M-PBS | 0.058 |

**Table 3. Correspondence of number**

| Number in the present application | Unique sequence | Number in the present application | Unique sequence |
|---|---|---|---|
| MHAB14-1 | M26H2-3012 | MHAB14-14 | M26H2-2002 |
| MHAB 14-2 | M26H2-3088 | MHAB14-15 | M26H2-2089 |
| MHAB 14-3 | M26H2-3176 | MHAB14-16 | M26H2-2048 |
| MHAB14-4 | M26H2-3016 | MHAB14-17 | M26H2-2152 |
| MHAB14-5 | M26H2-3038 | MHAB14-18 | M26H2-2032 |
| MHAB14-6 | M26H2-3011 | MHAB14-19 | M26H2-2179-4 |
| MHAB14-7 | M26H2-3027 | MHAB14-20 | M26H2-2168 |
| MHAB14-8 | M26H2-3015 | MHAB14-21 | M26H2-2073 |
| MHAB14-9 | M26H2-3050 | MHAB14-22 | M26H2-2141 |
| MHAB14-10 | M26H2-3134 | MHAB14-23 | M26H2-2109 |
| MHAB14-11 | M26H2-3056 | MHAB14-24 | M26H2-2150 |
| MHAB14-12 | M26H2-2001 | MHAB14-25 | M26H2-2081 |
| MHAB14-13 | M26H2-2030 | MHAB14-26 | M26H2-2087 |

### Example 2. Detection of anti-human HER2 nanobody activity

### 2-1 ELISA binding assay

The binding ability of the MHAB 14 candidate antibody (anti-human HER2 VHH-Fc fusion protein) to human HER2 antigen was detected using ELISA. The Her2/ERBB2 Protein, Human, Recombinant (ECD, His Tag) antigen (Sinobiological, Cat. No. 10004-H08H) was diluted to 0.5 µg/mL with PBS, added to an ELISA plate (Corning, Cat. No. 9018) at 100 µL/well, and coated overnight at 2°C to 8°C. The plate was washed three times with PBST, blocked with 1% BSA/PBST at room temperature for 1 hour, and then washed five times with PBST. The test antibody (the number of the test antibody was MHAB14, the positive control antibody was Trastuzumab; the initial concentration was 10 µg/mL, serial 7-fold dilution was performed) was added, incubated for 1 hour, and then the plate was washed seven times with PBST to remove unbound antibodies. Goat Anti-Human IgG Fc (HRP) (Abcam, Cat. No. ab97225), diluted at 1:50,000, was added and incubated at room temperature for 30 minutes. After removing the excess secondary antibody, 1-Step^{™} Ultra TMB-ELISA Substrate Solution (Absin, Cat. No. 9178) was added at 100 µL/well, and the plate was incubated for color development at room temperature in the dark for 10 minutes. Then, 100 µL of TMB Stop Solution (Absin, Cat. No. abs9472) was added to stop the reaction. Absorbance at 450 nm was read using a microplate reader, and a four-parameter curve was plotted using GraphPad.

All experimental results are expressed as mean + SEM (standard error of the mean). plotting and data analysis were performed using Prism 6 (GraphPad) software.

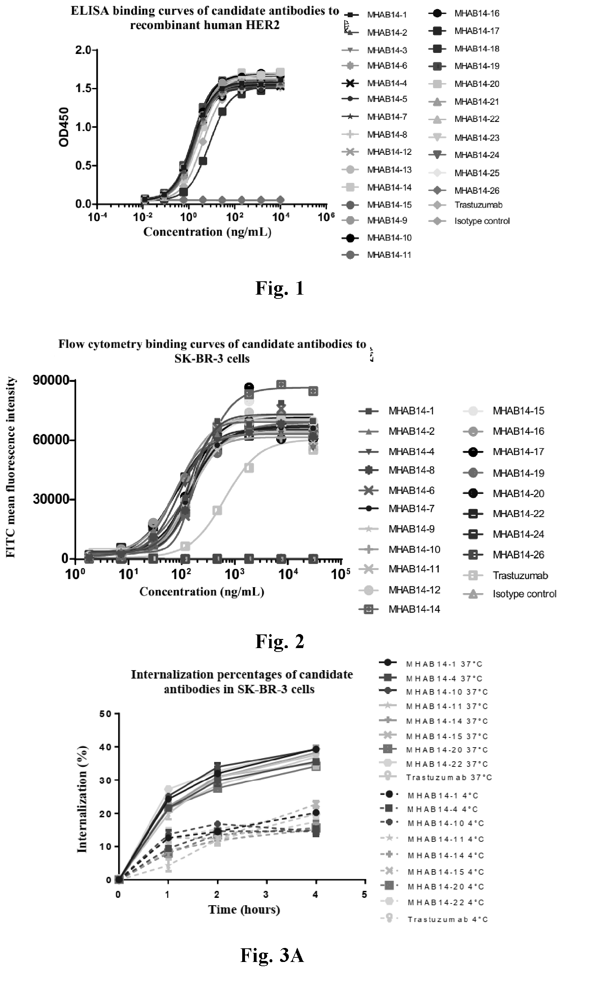

The results are shown in Fig. 1 and Table 4. The binding activity of the MHAB 14 candidate antibody to human HER2 antigen was as follows: except for MHAB14-18 and MHAB14-26, the EC₅₀ values of all other sequences were lower than that of the positive control Trastuzumab.

**Table 4. EC₅₀ results**

| Ab | MHAB 14-1 | MHAB 14-2 | MHAB 14-3 | MHAB 14-4 | MHAB 14-5 | MHAB 14-6 | MHAB 14-7 | MHAB 14-8 | MHAB 14-9 |
|---|---|---|---|---|---|---|---|---|---|
| bottom | 0.04436 | 0.0508 | 0.04783 | 0.04942 | 0.05478 | 0.05007 | 0.05335 | 0.04675 | 0.04201 |
| Top | 1.586 | 1.573 | 1.569 | 1.555 | 1.505 | 1.569 | 1.537 | 1.505 | 1.677 |
| EC₅₀(ng/ mL) | 1.557 | 1.78 | 1.993 | 1.861 | 1.692 | 2.126 | 1.602 | 1.659 | 1.809 |

**Continued from Table 4. EC₅₀ results**

| Ab | MHAB 14-10 | MHAB 14-11 | MHAB 14-12 | MHAB 14-13 | MHAB 14-14 | MHAB 14-15 | MHAB 14-16 | MHAB 14-17 | MHAB 14-18 |
|---|---|---|---|---|---|---|---|---|---|
| bottom | 0.05164 | 0.05189 | 0.053 | 0.04316 | 0.03073 | 0.05209 | 0.03982 | 0.04911 | 0.04617 |
| Top | 1.684 | 1.606 | 1.575 | 1.572 | 1.68 | 1.529 | 1.518 | 1.551 | 1.519 |
| EC₅₀(ng/ mL) | 1.764 | 1.752 | 1.78 | 1.906 | 3.014 | 1.837 | 1.74 | 1.836 | 7.876 |

**Continued from Table 4. EC₅₀ results**

| Ab | MHAB 14-19 | MHAB 14-20 | MHAB 14-21 | MHAB 14-22 | MHAB 14-23 | MHAB 14-24 | MHAB 14-25 | MHAB 14-26 | Trastuzu mab |
|---|---|---|---|---|---|---|---|---|---|
| bottom | 0.06296 | 0.05487 | 0.05636 | 0.06074 | 0.0596 | 0.06232 | 0.0549 | 0.05622 | 0.04812 |
| Top | 1.667 | 1.706 | 1.681 | 1.633 | 1.664 | 1.618 | 1.658 | 0.0589 | 1.576 |
| EC₅₀(ng/ mL) | 1.488 | 1.852 | 1.81 | 1.726 | 1.947 | 1.828 | 2 | N/A | 4.244 |

### 2-2 FACS binding assay

In the FACS assay, the binding of the candidate antibodies was detected using human breast cancer SK-BR-3 cells endogenously expressing human HER2 (the complete culture medium for SK-BR-3 cells consisted of ATCC-formulated McCoy's 5a Medium Modified, Catalog No. 30-2007+10% FBS, Sigma-Aldrich, Cat. No. F8687+1% P/S, Gibco, Cat. No. 15140-122). The test antibodies were serially diluted (30000 ng/mL, 7500 ng/mL, 1875 ng/mL, 468.8 ng/mL, 117.2 ng/mL, 29.3 ng/mL, 7.3 ng/mL, and 1.8 ng/mL), and the binding of the test antibodies was detected using 100 µL of FITC anti-human IgG Fc Antibody (Biolegend, Cat. No. 366916) as the secondary antibody. FITC fluorescence intensity (Mean Fluorescence Intensity, MFI) was detected by flow cytometry.

All experimental results are presented as mean ± SEM (standard error of the mean). plotting and data analysis were performed using Prism 6 (GraphPad) software.

The FACS binding results are shown in Fig. 2. Except for MHAB 14-26, the EC₅₀ values for binding of the MHAB14 candidate antibodies to SK-BR-3 cells were all lower than that of Trastuzumab.

### 2-3 Internalization assay

Internalization activity assay was performed using human breast cancer SK-BR-3 cells that endogenously express human HER2. A cell suspension at 1×10⁶ cells/mL and test anti-HER2 antibody solutions at 10 µg/mL were prepared using FACS buffer (PBS containing 2% FBS). The cell suspension was added to 1.5 mL EP tubes and centrifuged at 1200 rpm for 3 minutes at 4°C, after which the supernatant was discarded. 1.5 mL of the test antibody solutions were individually added to the cells, followed by incubation on ice for 30 minutes. The cells were then washed four times with pre-chilled FACS buffer at 200 µL/well. The cells were resuspended in 1.5 mL of complete medium, mixed, and added to a 96-well plate at 100 µL/well. The plate was incubated respectively at 4°C and 37°C for 0 h, 1 h, 2 h, and 4 h (in duplicate). After incubation, the plate was centrifuged at 1200 rpm for 3 minutes at 4°C. 100 µL of diluted FITC anti-human IgG Fc Antibody (Biolegend, Cat. No. 366916) was added, and the cells were incubated at 4°C for 30 minutes. After washing away unbound secondary antibody, the FITC fluorescence intensity (Mean Fluorescence Intensity, MFI) was detected using flow cytometry. The internalization rate was calculated using the following formula: $Internalization \left(%\right) = 100 % - {MFI}_{T} / {MFI}_{T 0} \times 100 %$

All experimental results are presented as mean ± SEM (standard error of the mean). GraphPad Prism 6 (GraphPad Software) was used for plotting and data analysis.

The internalization percentage curves over time are shown in Fig. 3A. At 37°C, the MHAB14 candidate antibodies exhibited good internalization activity in SK-BR-3 cells (with MHAB14-10 showing the best internalization activity), and the internalization activity was comparable to that of Trastuzumab.

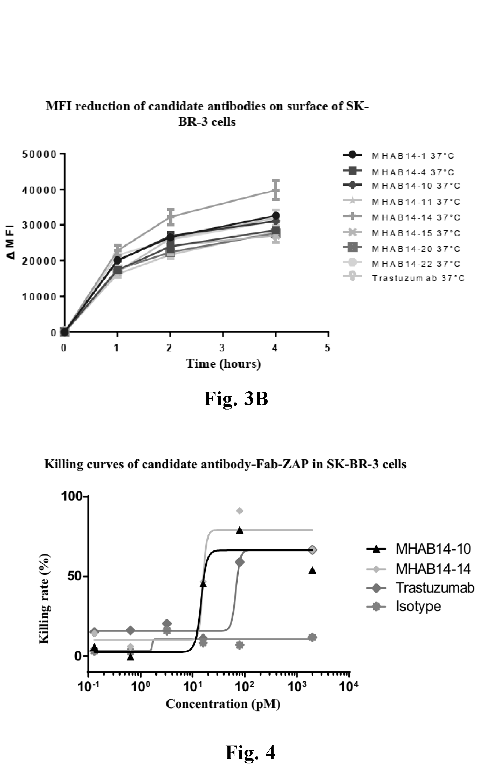

The MFI reduction curves of the candidate antibodies on the surface of SK-BR-3 cells are shown in Fig. 3B. The results indicated that at 37°C, MHAB14-14 showed the largest MFI reduction during internalization in SK-BR-3 cells (i.e., the absolute number of internalized antibodies in SK-BR-3 cells was the highest).

### 2-4 Fab-ZAP assay for detection of antibody internalization-mediated cell killing

Human breast cancer SK-BR-3 cells were digested and then resuspended in complete culture medium (ATCC-formulated McCoy's 5a Medium Modified, Catalog No. 30-2007+10% FBS, Sigma-Aldrich, Cat. No. F8687+1% P/S, Gibco, Cat. No. 15140-122). The cell density was adjusted to 4E4 cells/mL, and 50 µL of the cell suspension was added to each well of a 96-well cell culture plate. The plate was then incubated in a 37°C cell culture incubator for 16 hours. The next day, a ZAP dilution solution containing 9 nM ZAP-Fab was prepared using the culture medium. Subsequently, the antibody was serially diluted with the ZAP dilution solution to create working solutions ranging from 4000 pM to 0.26 pM (7 concentration gradients, 1:5 dilution). After incubation at 37°C for 15 minutes, the working solutions were added to the cell culture plate, 50 µL per well. After thoroughly mixing, the cell culture plate was placed in a 37°C cell culture incubator and incubated for an additional 120 hours. For the positive control, 7.5 µL of Triton-X 100 was added in advance to wells without antibody-Fab-ZAP treatment, followed by a 30-minute incubation. Wells without antibody-Fab-ZAP or Triton-X 100 treatment (NT) served as the negative control. Afterward, 20 µL of MTS (Promega, Cat: G3598B) was added to each well of the cell culture plate, and the plate was incubated in a 37°C cell culture incubator for 2 hours. The plate was then shaken for 10 seconds in a microplate reader, and the absorbance was measured at a wavelength of A490. The cell killing efficiency was calculated using the formula: killing rate % = 100% - (ODₛₐₘₚₗₑ - OD_{Triton-X100}) / (OD_{NT} - OD_{Triton-X100}) * 100%.

All experimental results are expressed as mean ± SEM (standard error of the mean). Prism 6 (GraphPad) software was used for plotting and data analysis.

The internalization-mediated cell killing curve is shown in Fig. 4. MHAB14-10\MHAB14-14-Fab-ZAP showed good killing activity against SK-BR-3 cells. Based on the combined FACS binding and internalization results, MHAB14-10 and MHAB14-14 would be humanized and further screened.

Wherein, the VHH sequence of MHAB14-10 is set forth in SEQ ID NO: 15, the CDR1 to CDR3 sequences defined by Kabat are set forth in SEQ ID NOs: 1, 2 and 4, respectively, and the CDR1 to CDR3 sequences defined by IMGT are set forth in SEQ ID NOs: 5-7, respectively. Wherein, the VHH sequence of MHAB14-14 is set forth in SEQ ID NO: 17, the CDR1 to CDR3 sequences defined by Kabat are set forth in SEQ ID NOs: 8, 9 and 11, respectively, and the CDR1 to CDR3 sequences defined by IMGT are set forth in SEQ ID NOs: 12-14, respectively.

### 2-5 Competitive ELISA binding assay

The ability of the candidate antibodies MHAB14-10 and MHAB14-14 to competitively bind to human HER2 antigen with Trastuzumab or Pertuzumab was detected using ELISA method. Trastuzumab and Pertuzumab were diluted to 0.3 µg/mL and 1 µg/mL, respectively, with PBS, added to an ELISA plate (Corning, Cat. No. 9018) at 100 µL/well, and coated overnight at 2°C to 8°C. The plate was washed three times with PBST, blocked with 1% BSA/PBST at room temperature for 1 hour, and then washed five times with PBST. The Her2/ERBB2 Protein, Human, Recombinant (ECD, His Tag) antigen (Sinobiological, Cat. No. 10004-H08H) was diluted to 0.4 µg/mL with 1% BSA/PBST. MHAB14-10, MHAB14-14, and the positive control antibodies Trastuzumab or Pertuzumab were diluted to 60 µg/mL and subjected to 3-fold serial dilution. The diluted MHAB14-10 and MHAB14-14 antibodies were added first, followed by 50 µL of diluted HER2, and diluted His Tag antigen, for a total volume of 100 µL/well, and incubated at room temperature for 60 minutes. After incubation, the plate was washed 7 times with PBST to remove unbound antibodies, followed by the addition of 1:12,000 diluted Anti-His tag Antibody (HRP), Mouse Monoclonal (Sino Biological, Cat. No. 105327-MM02T-H), and incubation at room temperature for 30 minutes. After washing away excess secondary antibody, 1-Step^{™} Ultra TMB-ELISA Substrate Solution (Absin, Cat. No. 9178) was added at 100 µL/well, and the plate was incubated at room temperature in the dark for 10 minutes for color development. The reaction was then stopped by adding 100 µL of TMB Stop Solution (Absin, Cat. No. abs9472). Absorbance values were read at 450 nm using a microplate reader, and four-parameter curves were plotted using GraphPad.

All experimental results are expressed as mean ± SEM (standard error of the mean). Prism 6 (GraphPad) software was used for plotting and data analysis.

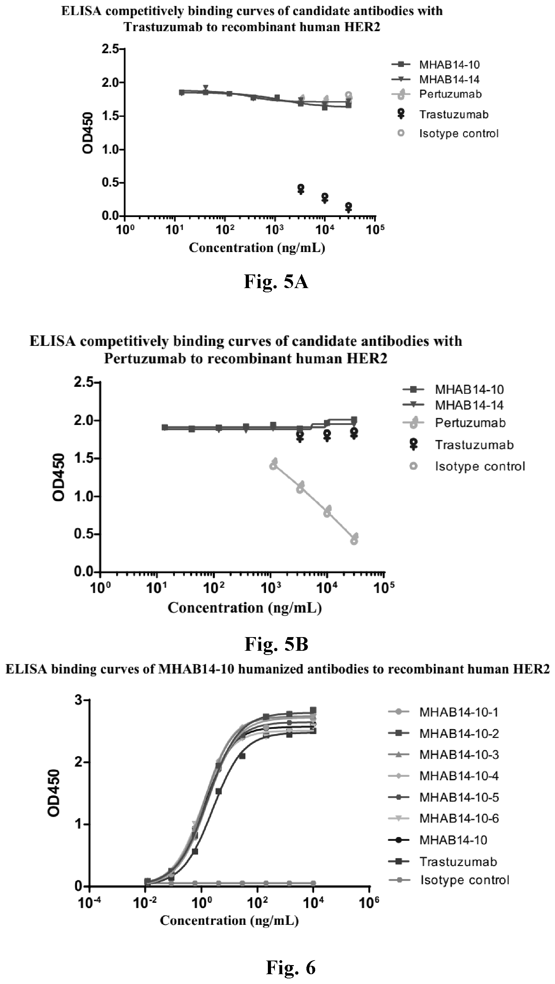

ELISA binding results are shown in Fig. 5A and Fig. 5B. Neither MHAB14-10 nor MHAB14-14 competitively bound to the HER2 antigen with Trastuzumab or Pertuzumab.

### Example 3. Humanization and activity detection of anti-human HER2 nanobodies

The VHH sequences of MHAB14-10 and MHAB14-14 were humanized to obtain two humanized nanobodies: MHAB14-10-5 (VHH as set forth in SEQ ID NO: 16) and MHAB14-14-6 (VHH as set forth in SEQ ID NO: 18). The VHH sequences were integrated into the Fc sequence (SEQ ID NO: 20) of human IgG1, and purified for expression in subsequent activity experiments.

### 3-1 ELISA binding assay

The binding capacity of the humanized sequences of MHAB14-10 and MHAB14-14 to human HER2 antigen was detected using ELISA method. The Her2/ERBB2 Protein, Human, Recombinant (ECD, His Tag) antigen (Sinobiological, Cat. No. 10004-H08H) was diluted to 0.5 µg/mL with PBS, added to an ELISA plate (Corning, Cat. No. 9018) at 100 µL/well, and coated overnight at 2°C to 8°C. The plate was washed 3 times with PBST, blocked with 1% BSA/PBST at room temperature for 1 hour, and then washed 5 times with PBST. The MHAB14-10 and MHAB14-14 humanized antibodies (antibody codes: MHAB14-10-1 to MHAB14-10-6 and MHAB14-14-1 to MHAB14-14-8, respectively; positive control antibody: Trastuzumab, with an initial concentration of 10 µg/mL, and subjected to 7-fold gradient dilution). After incubation for 1 hour, the plate was washed 7 times with PBST to remove unbound antibodies, followed by the addition of Goat Anti-Human IgG Fc (HRP) (Abcam, Cat. No. ab97225) diluted at 1:50,000, and incubated at room temperature for 30 minutes. After removing excess secondary antibody, 1-Step^{™} Ultra TMB-ELISA Substrate Solution (Absin, Cat. No. 9178) was added at 100 µL/well, and the plate was incubated at room temperature in the dark for 15 minutes for color development. The reaction was then stopped by adding 100 µL of TMB Stop Solution (Absin, Cat. No. abs9472). Absorbance at 450 nm was read using a microplate reader, and four-parameter curves were plotted using GraphPad.

All experimental results are expressed as mean ± SEM (standard error of the mean). Prism 6 (GraphPad) software was used for plotting and data analysis.

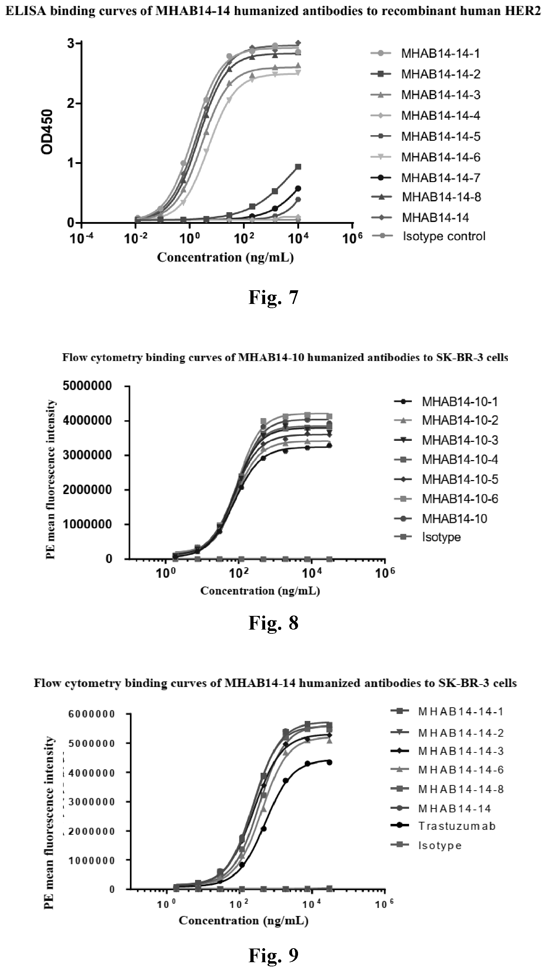

According to the ELISA binding results, the binding activities of the MHAB 14-10 humanized antibodies to the human HER2 antigen were comparable to that of the parental MHAB14-10 (Fig. 6 and Table 5). The affinities of the humanized sequences MHAB 14-14-2, MHAB 14-14-4, MHAB14-14-5, and MHAB14-14-7 for HER2 were significantly reduced compared to that of the parental MHAB 14-14, while the other sequences showed comparable activity (Fig. 7 and Table 6).

**Table 5. EC₅₀ results**

| Ab | MHAB14 -10-1 | MHAB1 4-10-2 | MHAB14 -10-3 | MHAB1 4-10-4 | MHAB14 -10-5 | MHAB14 -10-6 | MHAB1 4-10 | Trastuzu mab | Isotype control |
|---|---|---|---|---|---|---|---|---|---|
| bottom | 0.003229 | 0.01163 | 0.02740 | 0.03107 | 0.01089 | 0.01117 | 0.01953 | 0.01167 | N/A |
| Top | 2.721 | 2.801 | 2.743 | 2.742 | 2.650 | 2.513 | 2.575 | 2.482 | N/A |
| EC₅₀ (ng/mL) | 1.653 | 1.696 | 1.308 | 1.290 | 1.466 | 1.004 | 1.193 | 2.575 | N/A |

**Table 6. EC₅₀ results**

| Ab | MHAB1 4-14-1 | MHAB 14-14-2 | MHAB 14-14-3 | MHAB 14-14-4 | MHAB 14-14-5 | MHAB 14-14-6 | MHAB 14-14-7 | MHAB 14-14-8 | MHAB1 4-14 | Isotyp e control |
|---|---|---|---|---|---|---|---|---|---|---|
| bottom | 0.02584 | 0.0450 3 | 0.03630 | 0.05205 | 0.05103 | 0.02886 | 0.04843 | 0.02598 | 0.01802 | N/A |
| Top | 2.927 | 1.703 | 2.603 | 0.09980 | ~ 27.47 | 2.495 | 1.613 | 2.834 | 2.970 | N/A |
| EC₅₀ (ng/mL) | 1.406 | N/A | 2.857 | N/A | N/A | 4.972 | N/A | 2.207 | 2.006 | N/A |

### 3-2 FACS binding assay

In the FACS assay, the binding of MHAB14-10 and MHAB14-14 humanized antibodies was assessed using human breast cancer SK-BR-3 cells that endogenously express human HER2. The test antibodies were serially diluted (30000 ng/mL, 7500 ng/mL, 1875 ng/mL, 469 ng/mL, 117 ng/mL, 29 ng/mL, 7 ng/mL, and 1.8 ng/mL). The binding of the test antibodies was detected using the Goat anti-Human IgG Fc Secondary Antibody (Invitrogen, Cat. No. 12-4998-82), and the fluorescence intensity (Mean Fluorescence Intensity, MFI) of the bound secondary antibody was measured by flow cytometry.

All experimental results are expressed as mean ± SEM (standard error of the mean). Prism 6 (GraphPad) software was used for plotting and data analysis.

The binding curves are shown in Fig. 8 and Fig. 9. According to the FACS binding results, the binding activities of the MHAB14-10 humanized antibodies to SK-BR-3 cells were comparable to that of the parental MHAB14-10 (Fig. 8). The affinity of the humanized sequence MHAB14-14-2 for HER2 was significantly reduced compared to that of the parental MHAB14-14 (consistent with the ELISA results), while the other sequences showed affinity comparable to that of the parental MHAB14-14 (Fig. 9).

### 3-3 Internalization assay

Internalization activity assay was performed using human breast cancer SK-BR-3 cells that endogenously express human HER2. A cell suspension at 1×10⁶ cells/mL and test anti-HER2 antibody solutions at 10 µg/mL were prepared using FACS buffer (PBS containing 2% FBS). The cell suspension was added to 1.5 mL EP tubes and centrifuged at 1200 rpm for 3 minutes at 4°C, after which the supernatant was discarded. 1.5 mL of the test antibody solutions were individually added to the cells, followed by incubation on ice for 30 minutes. The cells were then washed four times with pre-chilled FACS buffer at 200 µL/well. The cells were resuspended in 1.5 mL of complete medium, thoroughly mixed, and added to a 96-well plate at 100 µL/well. The plate was incubated respectively at 4°C and 37°C for 0 h, 1 h, 2 h, and 4 h (in duplicate). After incubation, the plate was centrifuged at 1200 rpm for 3 minutes at 4°C. 100 µL of diluted FITC anti-human IgG Fc Antibody (Biolegend, Cat. No. 366916) was added, and the cells were incubated at 4°C for 30 minutes. After washing away unbound secondary antibody, the FITC fluorescence intensity (Mean Fluorescence Intensity, MFI) was detected using flow cytometry. The internalization rate was calculated using the following formula: $Internalization \left(%\right) = 100 % - {MFI}_{T} / {MFI}_{T 0} \times 100 %$

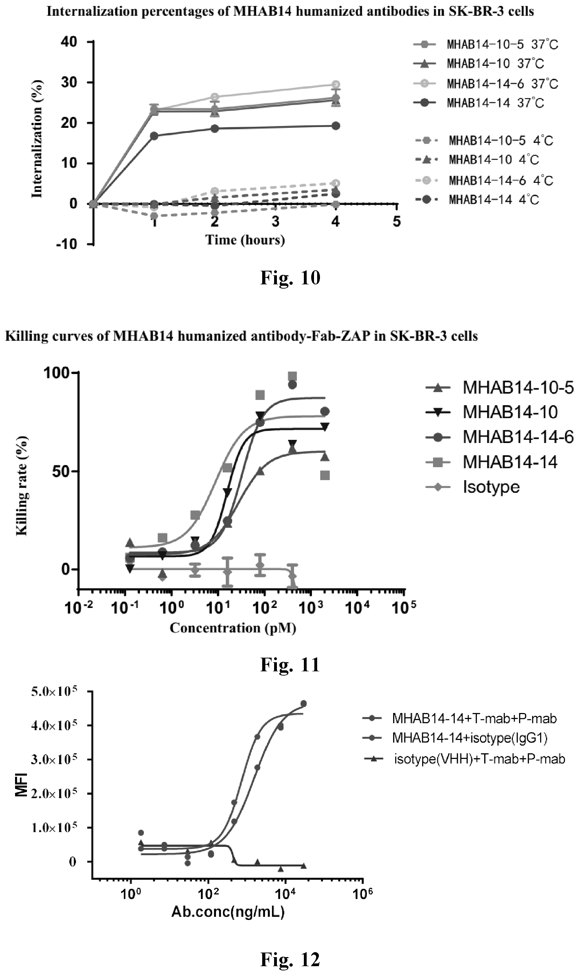

The internalization percentage curves are shown in Fig. 10. The internalization results indicated that, at 37°C, the humanized antibodies MHAB14-10-5 and MHAB14-14-6 exhibited good internalization activity in SK-BR-3 cells, comparable to that of the parental MHAB14-10 and MHAB14-14.

### 3-4 Fab-ZAP assay for detection of antibody internalization-mediated cell killing

Human breast cancer SK-BR-3 cells were digested and then resuspended in complete culture medium (ATCC-formulated McCoy's 5a Medium Modified, Catalog No. 30-2007+10% FBS, Sigma-Aldrich, Cat. No. F8687+1% P/S, Gibco, Cat. No. 15140-122). The cell density was adjusted to 4E4 cells/mL, and 50 µL of the cell suspension was added to each well of a 96-well cell culture plate. The plate was then incubated in a 37°C cell culture incubator for 16 hours. The next day, a ZAP dilution solution containing 9 nM ZAP-Fab was prepared using the culture medium. Subsequently, the antibody was serially diluted with the ZAP dilution solution to create working solutions ranging from 4000 pM to 0.26 pM (7 concentration gradients, 1:5 dilution). After incubation at 37°C for 15 minutes, the working solutions were added to the cell culture plate, 50 µL per well. After thoroughly mixing, the cell culture plate was placed in a 37°C cell culture incubator and incubated for an additional 120 hours. For the positive control, 7.5 µL of Triton-X 100 was added in advance to wells without antibody-Fab-ZAP treatment, followed by a 30-minute incubation. Wells without antibody-Fab-ZAP or Triton-X 100 treatment (NT) served as the negative control. Afterward, 20 µL of MTS (Promega, Cat: G3598B) was added to each well of the cell culture plate, and the plate was incubated in a 37°C cell culture incubator for 2 hours. The plate was then shaken for 10 seconds in a microplate reader, and the absorbance was measured at a wavelength of A490. The cell killing efficiency was calculated using the formula: killing rate % = 100% - (ODₛₐₘₚₗₑ - OD_{Triton-X100}) / (OD_{NT} - OD_{Triton-X100}) * 100%.

All experimental results are expressed as mean ± SEM (standard error of the mean). Prism 6 (GraphPad) software was used for plotting and data analysis.

The internalization-mediated cell killing curves are shown in Fig. 11. MHAB14-10-5-Fab-ZAP and MHAB14-14-6-Fab-ZAP both exhibited superior killing activity against SK-BR-3 cells.

### Example 4. Competitive FACS binding assay

The competitive binding capacity of the candidate antibody MHAB14-14 with Trastuzumab or Pertuzumab to the cell surface HER2 antigen was assessed using FACS on human breast cancer SK-BR-3 cells endogenously expressing HER2. Wherein, SK-BR-3 cells were cultured in complete SK-BR-3 cell culture medium (ATCC-formulated McCoy's 5a Medium Modified, Catalog No. 30-2007+10% FBS, Sigma-Aldrich, Cat. No. F8687+1% P/S, Gibco, Cat. No. 15140-122). A cell suspension (2.0×10⁶ cells/mL) and antibody dilutions were prepared with FACS buffer (PBS containing 2% FBS). Trastuzumab (final concentration 7.5 µg/mL) and Pertuzumab (final concentration 7.5 µg/mL) or Isotype control (final concentration 7.5 µg/mL) were added first, followed by the addition of serial dilutions of the candidate antibody MHAB14-14 or Isotype control (final concentration gradients: 30000 ng/mL, 7500 ng/mL, 1875 ng/mL, 468.8 ng/mL, 117.2 ng/mL, 29.3 ng/mL, 7.3 ng/mL, and 1.8 ng/mL). The mixture was incubated on ice at 4°C for 60 minutes, after which excess primary antibody was removed by washing with FACS buffer. Binding of the test antibodies was detected using 100 µL of diluted MonoRab^{™} Rabbit Anti-Camelid VHH Cocktail [PE] (GenScript, Cat. No. A02018) secondary antibody (diluted in PBS buffer containing 2% FBS at a ratio of 1:200). PE fluorescence intensity (Mean Fluorescence Intensity, MFI) was detected by flow cytometry.

All experimental results are expressed as mean ± SEM (standard error of the mean). Prism 6 (GraphPad) software was used for plotting and data analysis.

The binding curves are shown in Fig. 12. The FACS binding results indicate that under saturated concentrations of Trastuzumab and Pertuzumab, MHAB14-14 could still bind to HER2 antigen on the cell surface.

### Example 5. ELISA cross-species reactivity assay

The binding ability of MHAB14-14-6 (anti-human HER2 nanobody) to HER2 antigens from different species (human and cynomolgus) was detected using ELISA method. Human HER2, His Tag (ACRO, Cat. No. HE2-H5225) and Cynomolgus HER2 Protein, His Tag (Sino Biological, Cat. No. 90295-C08H) were diluted to 0.5 µg/mL with PBS, respectively, and added to an ELISA plate (Corning, Cat. No. 9018) at 100 µL/well, followed by overnight coating at 4°C. The plate was washed three times with PBST, and blocked with 3% BSA/PBST at 200 µL per well at room temperature for 1 hour. After the plate was washed 5 times with PBST, the test antibody was added at an initial concentration of 10 µg/mL and serially diluted 10-folds, followed by incubation at room temperature for 1 hour. The plate was then washed 7 times with PBST to remove unbound antibodies, and 100 µL of 1:50,000 diluted goat anti-human IgG Fc (HRP) (Abcam, Cat. No. ab97225) was added to each well, followed by incubation at room temperature for 30 minutes. After the plate was washed 5 times with PBST to remove excess secondary antibody, 100 µL of 1-Step^{™} Ultra TMB-ELISA Substrate Solution (Absin, Cat. No. 9178) was added to each well, and the plate was incubated at room temperature in the dark for 15 minutes for color development. The reaction was then stopped by adding 100 µL of TMB Stop Solution (Absin, Cat. No. abs9472) to each well. Absorbance was read at a wavelength of 450 nm using a microplate reader, and four-parameter logistic curves were plotted using GraphPad.

All experimental results are expressed as mean ± SEM (standard error of the mean). Prism (GraphPad) software was used for plotting and data analysis.

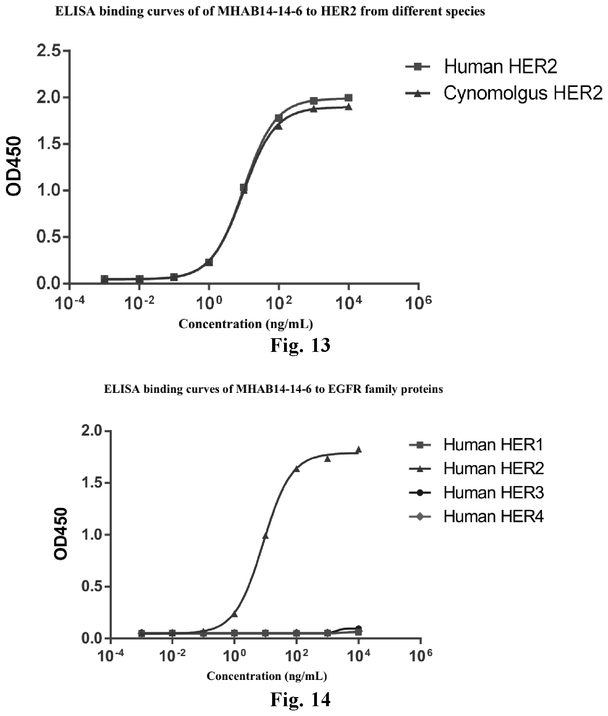

The results are shown in Fig. 13 and Table 7. MHAB14-14-6 exhibited specific binding to both human HER2 and cynomolgus HER2, with comparable binding activity.

**Table 7. Binding activity of MHAB14-14-6 to human HER2 and cynomolgus HER2**

| | Human HER2 | Cynomolgus HER2 |
|---|---|---|
| EC₅₀ (ng/ml) | 9.914 | 9.513 |

### Example 6. ELISA for EGFR family-specific binding

The binding capacity of MHAB14-14-6 (anti-human HER2 nanobody) to EGFR family proteins (HER1, HER2, HER3, and HER4) was detected using ELISA method. Human HER1, His Tag (Sino Biological, Cat. No. 10001-H08H); Human HER2, His Tag (ACRO, Cat. No. HE2-H5225); Human HER3 Protein, His Tag (ACRO, Cat. No. ER3-H5223); and Human HER4 Protein, His Tag (Sino Biological, Cat. No. 10363-H08H) were diluted to 0.5 µg/mL with PBS, respectively, and added to an ELISA plate (Corning, Cat. No. 9018) at 100 µL/well, followed by overnight coating at 4°C. After the plate was washed 3 times with PBST, 200 µL of 3% BSA/PBST was added to each well, and the plate was blocked at room temperature for 1 hour. The plate was washed 5 times with PBST, the test antibody was added at an initial concentration of 10 µg/mL and serially diluted 10-folds. After incubation at room temperature for 1 hour, the plate was washed 7 times with PBST to remove unbound antibodies. Then, 100 µL of goat anti-human IgG Fc (HRP) (Abcam, Cat. No. ab97225) diluted at 1:50,000 was added to each well, followed by incubation at room temperature for 30 minutes. After the plate was washed 7 times with PBST to remove excess secondary antibody, 100 µL of 1-Step^{™} Ultra TMB-ELISA Substrate Solution (Absin, Cat. No. 9178) was added to each well, and the plate was incubated at room temperature in the dark for 15 minutes for color development. The reaction was stopped by adding 100 µL of TMB Stop Solution (Absin, Cat. No. abs9472) to each well. Absorbance was read at a wavelength of 450 nm using a microplate reader, and four-parameter curves were plotted using Graphpad.

All experimental results are expressed as mean ± SEM (standard error of the mean). Prism (GraphPad) software was used for plotting and data analysis.

The results are shown in Fig. 14 and Table 8. MHAB14-14-6 bound only to HER2 and showed no binding activity to other members of the EGFR family, including HER1, HER3, and HER4. Therefore, the antibody of the present application exhibited outstanding selective binding activity to EGFR family proteins and specific binding activity to HER2.

**Table 8. Binding activity of MHAB14-14-6 to human HER1 to HER4**

| | Human HER1 | Human HER2 | Human HER3 | Human HER4 |
|---|---|---|---|---|
| EC₅₀ (ng/ml) | N/A | 8.45 | N/A | N/A |

Although specific embodiments of the present invention have been described in detail, those skilled in the art will understand that various modifications and variations can be made to the details based on all teachings disclosed, and all such changes modifications are within the scope of protection of the present invention. The entire scope of the present invention is given by the appended claims and any equivalents thereof.

## Claims

1. A nanobody or antigen-binding fragment thereof capable of specifically binding to human epidermal growth factor receptor-2 (HER2), wherein the nanobody or antigen-binding fragment thereof comprises: CDR1, CDR2 and CDR3 contained in VHH as set forth in any one of SEQ ID NOs: 15-18;
preferably, the CDRs are defined according to the Kabat, Chothia or IMGT numbering system.

2. The nanobody or antigen-binding fragment according to claim 1, which comprises:
(1) CDR1 as set forth in SEQ ID NO: 1; CDR2 as set forth in SEQ ID NO: 2 or 3; and CDR3 as set forth in SEQ ID NO: 4; wherein the CDRs are defined according to the Kabat numbering system;
(2) CDR1 as set forth in SEQ ID NO: 5; CDR2 as set forth in SEQ ID NO: 6; and CDR3 as set forth in SEQ ID NO: 7; wherein the CDRs are defined according to the IMGT numbering system;
(3) CDR1 as set forth in SEQ ID NO: 8; CDR2 as set forth in SEQ ID NO: 9 or 10; and CDR3 as set forth in SEQ ID NO: 11; wherein the CDRs are defined according to the Kabat numbering system;
(4) CDR1 as set forth in SEQ ID NO: 12; CDR2 as set forth in SEQ ID NO: 13; and CDR3 as set forth in SEQ ID NO: 14; wherein the CDRs are defined according to the IMGT numbering system.

3. The nanobody or antigen-binding fragment according to claim 1 or 2, wherein the nanobody or antigen-binding fragment comprises the sequence as set forth in SEQ ID NO: 15 or 17, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or more amino acids (e.g., a substitution, deletion, or addition of 1, 2, 3, 4, 5, 6, 7, or 8 amino acids) as compared thereto.

4. The nanobody or antigen-binding fragment according to claim 1 or 2, wherein the nanobody or antigen-binding fragment is humanized;
preferably, the nanobody or antigen-binding fragment further comprises a heavy chain framework region of a human immunoglobulin (e.g., a heavy chain framework region contained in the amino acid sequence encoded by a human heavy chain germline antibody gene), the heavy chain framework region optionally comprises one or more (e.g., 1, 2, 3, 4, 5, 6, 7, 8, 9, or 10) back mutations from human residues to camelid residues.

5. The nanobody or antigen-binding fragment according to claim 4, wherein the nanobody or antigen-binding fragment comprises the sequence as set forth in SEQ ID NO: 16 or 18, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion, or addition of one or more amino acids (e.g., a substitution, deletion, or addition of 1, 2, 3, 4, or 5 amino acids) as compared thereto.

6. A polypeptide construct capable of specifically binding to HER2, which comprises the nanobody or antigen-binding fragment according to any one of claims 1 to 5, and an immunoglobulin Fc domain;
preferably, the immunoglobulin Fc domain is optionally linked via a peptide linker to the N-terminus and/or C-terminus (e.g., C-terminus) of the nanobody or antigen-binding fragment;
preferably, the immunoglobulin Fc domain is a Fc domain of IgG, such as the constant region of a heavy chain of IgG1, IgG2, IgG3, or IgG4;
preferably, the immunoglobulin Fc domain comprises the sequence as set forth in SEQ ID NO: 19 or 20, or a sequence having a substitution, deletion, or addition of one or more amino acids, or any combination thereof (e.g., a sequence having a substitution, deletion, or addition of 1, 2, 3, 4, or 5 amino acids, or any combination thereof);
preferably, the polypeptide construct has a sequence as set forth in any one of SEQ ID NOs: 21-24.

7. An isolated nucleic acid molecule, which encodes the nanobody or antigen-binding fragment according to any one of claims 1 to 5 or the polypeptide construct according to claim 6.

8. A vector, which comprises the nucleic acid molecule according to claim 7; preferably, the vector is a cloning vector or an expression vector.

9. A host cell, which comprises the isolated nucleic acid molecule according claim 7 or the vector according to claim 8.

10. A method for preparing the nanobody or antigen-binding fragment thereof according to any one of claims 1 to 5 or the polypeptide construct according to claim 6, which comprises culturing the host cell according to claim 9 under a condition that allows protein expression, and recovering the nanobody or antigen-binding fragment thereof or the polypeptide construct from a culture of the cultured host cell.

11. A bispecific or multispecific antibody, which comprises the nanobody or antigen-binding fragment thereof according to any one of claims 1 to 5 or the polypeptide construct according to claim 6;
preferably, the bispecific or multispecific antibody is capable of specifically binding to HER2 and additionally specifically binding to one or more other targets;
preferably, the bispecific or multispecific antibody further comprises at least one second antibody having a second binding specificity against a second target.

12. A conjugate, which comprises the nanobody or antigen-binding fragment thereof according to any one of claims 1 to 5, the polypeptide construct according to claim 6, or the bispecific or multispecific antibody according to claim 11, and a conjugated portion connected thereto;
preferably, the conjugated portion is selected from therapeutic agents (e.g., cytotoxic agent, cytokine, toxin, or radionuclide);
preferably, the conjugate is an antibody-drug conjugate (ADC).

13. A pharmaceutical composition, which comprises the nanobody or antigen-binding fragment thereof according to any one of claims 1 to 5, the polypeptide construct according to claim 6, the isolated nucleic acid molecule according to claim 7, the vector according to claim 8, the host cell according to claim 9, the bispecific or multispecific antibody according to claim 11, or the conjugate according to claim 12, and a pharmaceutically acceptable carrier and/or excipient;
preferably, the pharmaceutical composition further comprises an additional pharmaceutically active agent, such as an antitumor agent.

14. A kit, which comprises the nanobody or antigen-binding fragment thereof according to any one of claims 1 to 5 or the polypeptide construct according to claim 6;
preferably, the kit comprises a conjugate, wherein the conjugate comprises the nanobody or antigen-binding fragment thereof according to any one of claims 1 to 5, or the polypeptide construct according to claim 6, and a detectable label that is linked to the nanobody or antigen-binding fragment thereof, or to the polypeptide construct; such as an enzyme (e.g., horseradish peroxidase or alkaline phosphatase), a chemiluminescent reagent (e.g., acridinium ester compound, luminol and derivative thereof, or ruthenium derivative), a fluorescent dye (e.g., fluorescein or fluorescent protein), a radionuclide, or biotin;
preferably, the kit comprises the nanobody or the antigen-binding fragment thereof according to any one of claims 1 to 5, or the polypeptide construct according to claim 6, and a second antibody capable of specifically recognizing the nanobody or the antigen-binding fragment thereof or the polypeptide construct; optionally, the second antibody further comprises a detectable label, such as an enzyme (e.g., horseradish peroxidase or alkaline phosphatase), a chemiluminescent reagent (e.g., acridinium ester compound, luminol and derivative thereof, or ruthenium derivative), a fluorescent dye (e.g., fluorescein or fluorescent protein), a radionuclide, or biotin.

15. A chimeric antigen receptor, which comprises the nanobody or antigen-binding fragment of any one according to claims 1 to 5 or the polypeptide construct according to claim 6;
preferably, the antigen-binding domain is expressed by an immune effector cell (e.g., a T cell).

16. An isolated nucleic acid molecule, which encodes the chimeric antigen receptor according to claim 15.

17. A vector, which comprises the isolated nucleic acid molecule according to claim 16; preferably, the vector is used for preparing a chimeric antigen receptor T cell.

18. A host cell, which comprises the isolated nucleic acid molecule according to claim 16 or the vector according to claim 17;
preferably, the host cell is an immune effector cell (e.g., a T cell or an NK cell);
preferably, the host cell is a chimeric antigen receptor T cell (CAR-T).

19. Use of the nanobody or antigen-binding fragment thereof according to any one of claims 1 to 5, the polypeptide construct according to claim 6, the isolated nucleic acid molecule according to claim 7, the vector according to claim 8, the host cell according to claim 9, the bispecific or multispecific antibody according to claim 11, the conjugate according to claim 12, or the pharmaceutical composition according to claim 13, or the kit according to claim 14, or the chimeric antigen receptor according to claim 15, in the manufacture of a medicament for prevention and/or treatment of a tumor in a subject;
preferably, the tumor expresses HER2;
preferably, the medicament is used to inhibit the growth of a tumor cell expressing HER2 and/or to kill the tumor cell;
preferably, the medicament further comprises an additional pharmaceutically active agent;
preferably, the additional pharmaceutically active agent is a drug with antitumor activity, such as an alkylating agent, a mitotic inhibitor, an antitumor antibiotic, an antimetabolite, a topoisomerase inhibitor, a tyrosine kinase inhibitor, a radionuclide, a radiosensitizer, an antiangiogenic agent, a cytokine, a molecularly targeted drug, an immune checkpoint inhibitor, or an oncolytic virus;
preferably, the tumor is selected from solid tumors, such as gastric cancer, lung cancer, liver cancer, head and neck cancer, skin cancer, colorectal cancer, ovarian cancer, esophageal cancer, pancreatic cancer, cervical cancer, mesothelioma, breast cancer, colorectal cancer, or oral squamous cell carcinoma;
preferably, the subject is a mammal, such as a human.

20. Use of the nanobody or antigen-binding fragment thereof according to any one of claims 1 to 5 or the polypeptide construct according to claim 6 in the manufacture of a kit for detecting whether a tumor can be treated by an HER2-targeted antitumor therapy, or for detecting the presence or amount of HER2 in a sample;
preferably, the sample is a cell sample (e.g., a sample containing tumor cells) or a body fluid sample (e.g., blood) from a subject (e.g., a mammal, such as a human).

21. A method for detecting the presence or amount of HER2 in a sample, which comprises using the nanobody or antigen-binding fragment thereof according to any one of claims 1 to 5 or the polypeptide construct according to claim 6;
preferably, the method is an immunological assay, such as Western blotting, enzyme immunoassay (e.g., ELISA), chemiluminescent immunoassay, fluorescence immunoassay, or radioimmunoassay.
